# EUROPEAN PATENT SPECIFICATION

(11) **EP 4 457 826 B1**
(45) Date of publication and mention of the grant of the patent: **23.04.2025**
(21) Application number: 23833043.5
(22) Date of filing: 14.12.2023
(51) Int. Cl.: G16H 10/40, G16H 40/63

(54) **TITRATION SUPPORT SYSTEM FOR COMPLICATED PATIENTS AND METHOD OF OPERATION THEREOF**
TITRATIONSUNTERSTÜTZUNGSSYSTEM FÜR KOMPLEXE PATIENTEN UND VERFAHREN ZUM BETRIEB DAVON
SYSTÈME DE SUPPORT DE TITRAGE POUR PATIENTS COMPLEXES ET SON PROCÉDÉ DE FONCTIONNEMENT

(30) Priority: 23.12.2022 US 202263434953 P
(43) Date of publication of application: 06.11.2024
(73) Proprietor: Koninklijke Philips N.V., 5656 AG Eindhoven (NL)
(72) Inventor: SHELLY, Benjamin Irwin, 5656 AG Eindhoven (NL); GOSWAMI, Tushar, 5656 AG Eindhoven (NL); BURLESON, Michael, 5656AG Eindhoven (NL)
(74) Representative: Philips Intellectual Property & Standards
(86) International application number: PCT/EP2023/085731
(87) International publication number: WO 2024/132822

(56) References cited:
- WO-A1-2022/058967
- WO-A2-02/43584
- US-A1- 2013 317 318
- US-A1- 2015 025 328
- US-A1- 2021 161 395
- DALAL APARNA: "Anesthesia for liver transplantation", TRANSPLANTATION REVIEWS, vol. 30, no. 1, 15 May 2015 (2015-05-15), pages 51 - 60, XP029351849, ISSN: 0955-470X, DOI: 10.1016/J.TRRE.2015.05.003
- MING-ZHER POH ET AL: "Continuous monitoring of electrodermal activity during epileptic seizures using a wearable sensor", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 4415 - 4418, XP032108053, ISBN: 978-1-4244-4123-5, DOI: 10.1109/IEMBS.2010.5625988

## Description

### FIELD OF THE PRESENT SYSTEM

The present system relates to a positive airway pressure (PAP) system including titration support for complicated patients and, more particularly, to a positive airway pressure (PAP) system including sensor-mediated control to support titration for complicated patients, and a method of thereof.

### BACKGROUND OF THE PRESENT SYSTEM

Sleep disordered breathing (SDB) is characterized by the disruption of normal respiratory patterns and ventilation during sleep. This sleep disorder phenotypically presents with many comorbidities such as cardiovascular hypertension, pulmonary hypertension, and congestive heart failure. Therapeutic efficacy has been defined with different methods, such as an adaptive support ventilation (ASV) method (where ASV machines deliver air pressure dynamically, adjusting according to the subject's breathing patterns), and continuous positive airway pressure (CPAP) method (where CPAP and machines deliver a constant level of air pressure) in disease sets such as obstructive sleep apnea (OSA) and the combination of OSA and central sleep apnea (CSA). Within these disease sets, congestive heart failure (CHF) has been shown to be a significant co-morbidity. However, positive air pressure (PAP) and ASV therapies, in large scale trials, have been shown to increase mortality in some CHF patients, especially those with reduced ejection fraction (HF-REF) who often suffer from poor clinical outcomes including hospitalization and mortality. This effect has been quantified in large-scale prospective trials such as the Canadian Continuous Positive Airway Pressure for Patients with Central Sleep Apnea and Heart Failure (CANPAP) trial and Predominate Central Sleep Apnea by Adaptive Servo Ventilation in Patients with Heart Failure (SERVE-HF) trial. These findings are also present alongside physiological improvements in short term settings such as heart rate variability (HRV), blood oxygenation, and various cardiac output measures. Due to this contradiction, more robust methods are required to treat sleep disorder breathing (SDB) in patients with CHF comorbidities to ensure cardiac output normality.

Current standard treatment of SDB requires PAP therapy. This therapy modality was first described in U.S. PAT. No. 4,944,310. In brief, PAP therapy pneumatically splints an airway through delivery of positive pressure (e.g., 4-20 cm H₂O). Air delivery to the airway is supplied by a motor-driven blower coupled to an air-delivery hose for gas transport to the nose/mouth encapsulated by a mask sealed by the patient's face. Other forms of PAP treatment utilize phenotypical more complicated methods such as: bi-level ventilation and servo-controlled ventilation for daily use before and during sleep.

Professional management of patients typically involves a robust signal set from a multitude of sensors collected only over a single night due to the constraints of the patient needing to be at a sleep/titration center together with a physician and/or clinician. Conventionally, parameters and modes of PAP machines are set by clinicians at the time of a single-night titration study, where air pressure delivered by the PAP machine is gradually increased until the PAP machine is calibrated to where the subject's optimum air pressure setting is determined when the breathing and sleep of patient or subject (receiving the delivered air via a patient interface such as a mask) becomes normal (e.g., within acceptable predetermined criteria). However, there is no robust and efficient current system to adapt PAP parameters to the patient's changing cardiac state since patients are not tested beyond the single night study (i.e., patients are not longitudinally monitored).

Home titration of CPAP machines can be a time and resource consuming process for both the patient and physician, especially in the cases of complicated patients, such as those with CHF. Clinically efficacious management of these patients currently requires comprehensive, multi-modal signal sets over a single night, as patients are again, not measured longitudinally. Home titration requires the schedules of both the patient and physician to align for the duration of an entire sleep study, requires a qualified sleep technician to monitor the study and adjust the CPAP machine as the study progresses, and also requires a dedicated sleep lab and equipment for the patient to sleep in comfort. All of these are obstacles and make CPAP titration more frustrating for the patient, more time consuming for the physician, and more expensive for all parties involved.

An inherent limitation of this method is its static nature. Titration occurs over a single night titration study, disallowing any adaptive, disease, context, and time-specific mode setting. Thus, any change in patient condition or adaptation to therapy requires re-titration in a clinical setting. Any of these obstacles standing in the way between a patient and their care can lower outcomes or even prevent treatment altogether.

Additionally, current practice contraindicates PAP therapy (especially ASV therapy) for the vast majority of heart failure with reduced ejection fraction (HFrEF) patients due to the inability to monitor and control for PAP-mediated risk factors that may increase the probability of mortality/morbidity. Thus, many heart failure with reduced ejection fraction (HFrEF) patients who would benefit from ASV therapy cannot currently receive it.

There is therefore a need for home titration including for complicated or at-risk congestive heart failure (CHF) patients to control risk factors for poor clinical outcomes that can actively monitor and manage with minimal therapeutic intervention.

To overcome the aforementioned barriers and detriments as well as others, there is a need for a system which can provide patients, including those who may have complicated comorbidities, use of PAP machines while mitigating the key PAP-mediated cardiac risk factors. In addition, there is a need to give access to PAP machines for patients that exhibit cardiac risk factors that also frees up resources for the physician to use on more complicated or at-risk patients, thus overcoming the aforementioned barriers and detriments as well as others of conventional systems.

PCT application WO2022/058967 A1 discloses a method including receiving physiological data associated with a user, and determining an emotion score associated with the user based at least in part on the physiological data. The method includes modifying a setting of a respiratory therapy system responsive to determining that the emotion score satisfies a predetermined condition. Article "Continuous Monitoring of Electrodermal Activity During Epileptic Seizures Using a Wearable Sensor", by Ming-Zher Poh, 32nd Annual International Conference of the IEEE EMBS, 2010, discloses a method for monitoring sympathetic nervous system activity during epileptic seizures using a wearable sensor measuring electrodermal activity (EDA). US patent application US 2021/0161395 A1 discloses a method including detecting, via a sensor, vibrations originating from a vein of a subject, and obtaining an intensity spectrum of the detected vibrations over a range of frequencies. The obtained intensity spectrum is used to determine a pulmonary capillary wedge pressure (PCWP) or a mean pulmonary arterial pressure.

### SUMMARY OF THE PRESENT SYSTEM

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a titration support system (100) for a positive air pressure (PAP) device, the system including: at least one controller (120, 190, 194); an external sensor (114) comprising at least an electrical impedance tomography (EIT) sensor, the external sensor (114) configured to: determine whether the EIT sensor is coupled to skin of a user; establish communication with the at least one controller (120, 190, 194) in response to determining that the external sensor (114) is coupled to the skin of the user; sense a physiological parameter of the user and form corresponding sensor information; determine whether cardiac output is diminished based upon the sensor information; and transmit the sensor information including an indication of whether the cardiac output is diminished to the at least one controller; the at least one controller (120, 190, 194) configured to: control a flow generator (144) to provide therapeutic air assist to the user; receive the sensor information from the external sensor; determine whether the sensor information comprises the indication that the cardiac output is diminished; begin auto titration to restore the cardiac output to homeopathic levels in response to determining that the cardiac output is diminished; identify a pressure sensitivity of the user based on trending of the cardiac output mapped as a function on a control parameter of the PAP device; and control the PAP device (102) to adapt PAP therapy based on the pressure sensitivity. The external sensor (114) may be further configured to determine mean pulmonary arterial pressure (MPAP). The external sensor (114) may determine that the cardiac output is diminished when the mean pulmonary arterial pressure (MPAP) is determined to be equal to or greater than 25 mm Hg.

The external sensor (114) may be further configured to sense sympathetic nervous system activity by measuring electrodermal activity, form corresponding sensor information, and transmit this sensor information to the at least one controller (120, 190, 194). The external sensor (114) may determine that the cardiac output is diminished when increased sympathetic nervous system activity is detected. The external sensor (114) may further include at least one of an electrocardiogram (ECG), a photoplethysmogram (PPG), a galvanic skin response (GSR), and a balistocardiogram (BCG) sensor. In accordance with embodiments, to restore the cardiac output to homeopathic levels, the at least one controller (120, 190, 194) is further configured control the flow generator (144) to decrease expiratory positive airway pressure (EPAP) by 1 cm H2O every threshold time period until the cardiac output is restored to the homeopathic levels. The at least one controller (120, 190, 194) may be further configured to determine the homeopathic levels during a titration period. The at least one controller (120, 190, 194) may be further configured to determine a maximum pressure PMAX of therapeutic air to assist.

In accordance with embodiments of the present system, there is disclosed a computer program, which, when executed by the controller of the titration support system, causes the titration support system to perform a method of providing titration support for a positive air pressure (PAP) device, the method including acts of: determining whether an electrical impedance tomography (EIT) sensor is coupled to skin of a user; establishing communication with at least one controller (120, 190, 194) in response to determining that the external sensor (114) is coupled to the skin of the user; sensing a physiological parameter of the user and forming corresponding sensor information; determining whether cardiac output is diminished based upon the sensor information; and transmitting the sensor information including an indication of whether the cardiac output is diminished to the at least one controller; controlling a flow generator (144) to provide therapeutic air assist to the user; receiving the sensor information from the external sensor; determining whether the sensor information comprises the indication that the cardiac output is diminished; beginning auto titration to restore the cardiac output to homeopathic levels in response to determining that the cardiac output is diminished; identifying a pressure sensitivity of the user based on trending of the cardiac output mapped as a function on a control parameter of the PAP device; and controlling the PAP device to adapt PAP therapy based on the pressure sensitivity. The method may include determining mean pulmonary arterial pressure (MPAP). The method may include determining that the cardiac output is diminished when the mean pulmonary arterial pressure (MPAP) is determined to be equal to or greater than 25 mm Hg. The method may include sensing sympathetic nervous system activity by measuring electrodermal activity, forming corresponding sensor information, and transmitting this sensor information to the at least one controller (120, 190, 194).

The method may include determining that the cardiac output is diminished when increased sympathetic nervous system activity is detected. The method may include, wherein the external sensor (114) further comprises at least one of an electrocardiogram (ECG), a photoplethysmogram (PPG), a galvanic skin response (GSR), and a balistocardiogram (BCG) sensor. In accordance with the method, to restore the cardiac output to homeopathic levels, the at least one controller (120, 190, 194) controlling the flow generator (144) to decrease expiratory positive airway pressure (EPAP) by 1 cm H2O every threshold time period until the cardiac output is restored to the homeopathic levels. The method may include, the at least one controller (120, 190, 194) determining the homeopathic levels during a titration period. The may include, the at least one controller (120, 190, 194) determining a maximum pressure PMAX of therapeutic air to assist.

In accordance with embodiments of the present system, there is disclosed a computer readable non-transitory medium having computer readable program code for operating on a computer for performing a method of providing titration support for a positive air pressure (PAP) device, the method including acts of: determining whether an electrical impedance tomography (EIT) sensor is coupled to skin of a user; establishing communication with at least one controller (120, 190, 194) in response to determining that the external sensor (114) is coupled to the skin of the user; sensing a physiological parameter of the user and forming corresponding sensor information; determining whether cardiac output is diminished based upon the sensor information; and transmitting the sensor information including an indication of whether the cardiac output is diminished to the at least one controller; controlling a flow generator (144) to provide therapeutic air assist to the user; receiving the sensor information from the external sensor; determining whether the sensor information comprises the indication that the cardiac output is diminished; beginning auto titration to restore the cardiac output to homeopathic levels in response to determining that the cardiac output is diminished; identifying a pressure sensitivity of the user based on trending of the cardiac output mapped as a function on a control parameter of the PAP device; and controlling the PAP device to adapt PAP therapy based on the pressure sensitivity.

### BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:
FIG. 1 illustratively shows a schematic block diagram of a portion of a system operating in accordance with embodiments of the present system;
FIG. 2 illustratively shows a functional flow diagram performed by a process for a CHF comorbid patient with SDB in accordance with embodiments of the present system;
FIG. 3 illustratively shows a functional flow diagram performed by a process for biosensor CPAP data migration in accordance with embodiments of the present system; and
FIG. 4 illustratively shows a functional flow diagram performed by a process for resolving diminished cardiac output in response to ASV therapy with SDB patients comorbid with CHF in accordance with embodiments of the present system;

### DETAILED DESCRIPTION OF THE PRESENT SYSTEM

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or," and formatives thereof, should be understood to mean that only one or more of: the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances." For example, the terms may indicate a deviation from the indicated numerical value(s) of ±20 %, ±15 %, ±10 %, ±5 %, ±1 % ±0.5 % or ±0.1 %.

The terms clinician, clinicians, and/or formatives thereof, may include a professional such as a doctor, a clinician, a technologist, an operator, an expert, medical specialist, a technician, and/or the like, who may operate and/or review information related to the patient such as system setting information (SSI), system information, and/or data generated by embodiments of the present system and associated data for review by a as a doctor, a clinician, a technologist, an operator, an expert, medical specialist, a technician, and/or the like unless the context indicates otherwise. It should be appreciated that the clinician may calibrate and/or set parameters of PAP devices operating in accordance with embodiments of the present system.

The terms patient, patients, and/or formatives thereof, may include patients or other individuals (e.g., persons, subjects, subjects-under-test, etc.) or other users who may be receiving any type of sleep apnea monitoring or assessment using systems, machines, processes, and/or methods operating in accordance with embodiments of the present system.

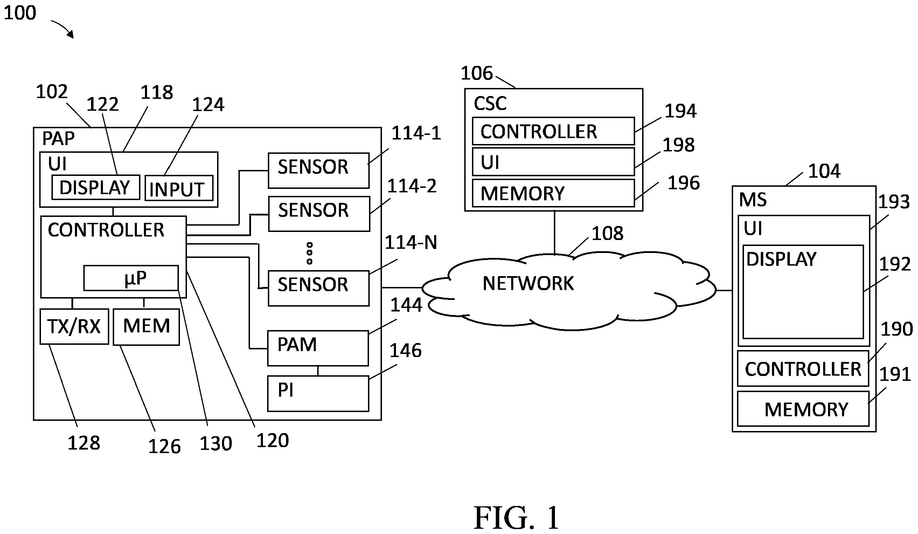

FIG. 1 illustratively shows a schematic block diagram of a portion of a system 100 (hereinafter the system 100) operating in accordance with embodiments of the present system. The system 100 may include one or more of a positive airway pressure (PAP) 102 device (hereafter PAP 102), a mobile station (MS) 104, and a clinical service center (CSC) 106, one or more of which may communicate with the other via a network 108, using any suitable communication method or methods such as wired, wireless, and/or optical communication methods.

The network 108 may include any suitable network such as an ad-hoc network, a Bluetooth^{™} network, a body area network (BAN), a personal area network (e.g., Bluetooth^{™}, etc.), a Zigbee network, Wi-Fi, a local area network (LAN), a wide area network (WAN), a telephony network, a cellular network (e.g., 5G, etc.), and/or combinations thereof. The PAP 102, the MS 104, the CSC 106, and the network 108 may be controlled by at least one controller which may control the overall operation of the system and may include one or more logic devices such as a microprocessor and/or the like.

The at least one controller may access at least one memory and may obtain system setting information (SSI) which may include one or more of operating parameters (e.g., scan type, initialization settings, etc.), operating settings, threshold values, warnings, display settings, user information, patient information such as patient identification (ID), content (e.g., video, audio, etc.), etc. The SSI may be set by the system and/or user and may be updated during use. For example, the SSI may be updated to reflect titration settings, etc., as they may be updated by the system and/or clinician. The at least one controller may generate and render an interface with which the patient and/or clinician may, for example, interact with to, for example, set, reset, select, and/or adjust one or more settings of the SSI and/or enter information (e.g., ID, test type, settings, parameters (e.g., test duration), results, etc.) which may be stored in the SSI in at least one memory of the system. The SSI may be different for each patient. The at least one controller may be a local and/or distributed throughout the system.

The MS 104 may include any suitable mobile station(s) such as a smart-phone (e.g., an I-Phone, a Galaxy phone, etc.), a personal digital assistant (PDA), a smart watch (e.g., an Apple^{™} Watch^{™}, a Galaxy^{™} watch, etc.), and/or the like, and may include a memory 191, at least one controller 190, a user interface (UI) 193, and a transceiver (Tx/Rx) configured to communicate with the network 108.

The UI 193 may provide an interface with which a user (e.g., the patient, the clinician, etc.) may interact, such interfaces may include, inter alia, a touch-screen display 192, an optical sensor such as a camera, a proximity sensor, a speaker, and/or a microphone. However, other user interfaces are also envisioned. The UI 193 may render information, such as content, etc., on the display and/or a speaker for the convenience of the patient or clinician, and may receive information entered by the patient or clinician such as selections, requests, commands, patient or clinician settings, etc., using any suitable input method such as gesture inputs, a touch input, voice input, etc. The memory 191, and/or other memories of the PAP 102 and/or the CSC 106, may store information for use by the system such as the SSI, sleep monitoring information (SMI), system settings, system parameters, operating parameters, and/or combinations thereof, as will be discussed below. The patient identification (ID) may be employed to identify the patient and the corresponding test results. The MS 104 may transmit information such as operating parameters, instructions, requests, commands (e.g., a synchronization command, etc.), to the PAP 102, and may receive information generated by the PAP 102 such as the SSI, operating parameters, sensor information, and/or combinations thereof, from the PAP 102 for further processing. The MS 104 may further receive and/or transmit sleep monitoring information (SMI), current settings, warnings, PAP status, etc., as discussed herein.

Data transfer may occur on a periodic or non-periodic intervals as may be set forth in the SSI and/or requested by the system (e.g., the PAP 102, the CSC 106, or the MS 104), patient, and/or clinician. For example, information may be transmitted from one or more of the PAP 102, the CSC 106, and the MS 104 to another of the PAP 102, the CSC 106, and the MS 104 (or internally within any of the PAP 102, the CSC 106, and the MS 104), in response to a query or request, and/or may be pushed at periodic or non-periodic intervals to a receiver depending upon system configuration and/or settings. Sensor information may be transferred directly or via, for example, the network 108, from one or more of the PAP 102, the CSC 106, and the MS 104 to another of the PAP 102, the CSC 106, and the MS 104, for further processing.

For the sake of clarity, only a single MS 104 will be discussed unless the context indicates otherwise. However, without limitation other MSs may be employed by the system. For example, a clinician such as a doctor may have a further MS in addition to the (primary) MS of the system 100, where one or more of the primary MS and the further MS may receive information such as SSI, SMI, etc., from other portions of the system for further review, processing, and/or storage. Accordingly, there may be a plurality of MSs 104 employed by the system 100 one or more of which may communicate with each other via the network 108 or any suitable communication link.

The CSC 106 may include one or more of a controller 194, a memory 196, and a UI 198. The controller 194 may control the overall operation of the CSC 106 and, as such may receive information, such as the SSI, SMI, and/or other information from the PAP 102 and/or MS 104 for further processing. The controller 194 may control operation of the system, if desired. However, it should be understood that any controller of the system may control operation of the system if desired, such as the controller 190 of the MS 104 and/or a controller 120 of the PAP 102. The CSC 106 may further communicate with the PAP 102 and/or MS 104 to, for example, transmit diagnostic results of the processing (e.g., operating mode(s), etc.) to be rendered on a rendering device of the system, such as on a rendering device of the MS 104, for the convenience of the user. The diagnostic results may be stored in the SMI in a memory of the system. The CSC 106 may process data in real time or may obtain data that has been stored in a memory of the system.

One or more controllers of the system 100 may employ machine learning and/or artificial intelligence (AI) engines to process information generated by the system such as sensor information, the SMI as well as data provided to the system such as historical information. The one or more controllers of the system 100 may be operative to monitor for and detect events, and form corresponding event information, etc., and/or may store and/or update results in a memory of the system (e.g., within the SMI), and may forward these results to the corresponding patient and/or clinician for their convenience as may be set forth, for example, in the SSI. The SMI may be accessed by one or more of the CSC 106, the MS 104, and the PAP 102 of the system for further review, processing, and/or analysis.

The PAP 102 may include one or more of a controller 120, a transceiver or transmitter/receiver (TX/RX) 128, a memory 126, sensors 114-1 through 114-N (generally sensors 114, where N is an integer), a pressure assist module (PAM) 144, a patient interface (PI) 146, and a user interface (UI) 118 with which a user, such as the patient or clinician, may interact. In accordance with embodiments, thought N sensors 114 are illustratively shown, more or less (e.g., 1, 2) may be utilized in accordance with the present system. The controller 120 may control the overall operation of the PAP 102 and may communicate with one or more of the CSC 106, the MS 104, and the network 108, via the TX/RX 128. The controller 120 may include one or more logic devices such as a microprocessor (µP) 130 and may control the overall operation of the PAP 102. It should be appreciated that in some embodiments the controller 120 may include digital and/or analog control circuitry. It is further envisioned that the PAP 102 and/or portions thereof, such as one or more of the sensor 114 may be a stand-alone unit with its own power supply as well as with its own controller to control sensor operations and/or make determinations from sensor information measured by the sensor. Accordingly, optional power supplies and/or charging systems (e.g., wired, wireless, solar, etc.) may be provided to provide energy to the corresponding power supply depending upon system configuration.

The UI 118 may include a display 122 (e.g., a touch-screen display) and a user input interface 124 which may be combined with, or separate from, each other. The user input interface 124 may include a keyboard, a mouse, a trackball, a touch-sensitive sensor, or other device, such as a touch-sensitive display, which may be stand alone or part of a system, such as part of a laptop, a personal digital assistant (PDA), a mobile phone (e.g., a smart phone), a smart watch, an e-reader, a monitor, a smart or dumb terminal or other device for communicating with the controller 120 via any operable link such as an optical, wired, and/or wireless communication link. The user input interface 124 may be operable for interacting with the controller 120 including enabling interaction within a UI 118 as described herein. Clearly the controller 120, the sensor 114, the user input interface 124, the user interface (UI) 118, the memory 126, the PAM 144, the PI 146, the CSC 106, the MS 104, and the network 108 may all or partly be a portion of a computer system or other device. The UI 118 may be operative to provide audio/visual feedback as well as graphical user interfaces (GUIs) to the user of the present system and may inform the operator of operating parameters, operating states, etc.

The controller 120 may be operative to render content, such as still or video information, as well as audio information on a rendering device of the system such as on the display 122 and/or speaker of the UI 118. This information may include information related to operating parameters, instructions, feedback, events, and/or other information related to an operation of the system, or portions thereof, such as SSI or portions thereof, SMI, application data, data generated by the system, operating parameters, etc., and/or combinations thereof. Clinical decision support (CDS) may be employed to analyze data generated by the system and may generate determinations (e.g., clinical decisions (CDs)) which may be stored in the system and/or rendered for the convenience of the patient and/or clinician depending upon settings in the SSI. For example, the CDs may be stored in the SMI and/or the SSI, where the SSI may include system setting information that may be used by the system so set operational parameters and settings as may be set by the system and/or user.

The controller 120 may be operable for providing control signals and/or performing operations in response to input signals from the user input device 124 as well as in response to other devices of a network, such as the sensor 114, and executing instructions stored in a memory of the system such as the memory 126. The µP 130 may include one or more of a microprocessor, an application-specific and/or general-use integrated circuit(s), a logic device, etc. Further, the µP 130 may be a dedicated processor for performing in accordance with the present system and/or may be a general-purpose processor wherein only one of many functions operates for performing in accordance with the present system. The µP 130 may operate utilizing a program portion, multiple program segments, and/or may be a hardware device utilizing a dedicated or multi-purpose integrated circuit. It is envisioned that one or more portions of the PAP 102, such as the controller 120, may be operationally coupled to the memory 126, the TX/RX 128, the user interface (UI) 118, the rendering device such as a display 122, the sensor 114, the user input interface 124, the PAM 144, the PI 146, and the network 108.

The memory 126 include any suitable memory configured to store information used by, or generated by, the PAP 102 such as operating instructions, the SSI, the SMI, etc. In some embodiments, the memory 126 may store information related operating conditions, such as operating modes, as well as PAP control parameters (e.g., EPAPmax, PSmax, Pmax, PSmin, EPAPmin, Target Tidal Volume, IPAPmin, IPAPmax, Backup Breath Rate, Backup inspiratory time, etc.) of the PAP 102. Operating modes, for example, may include CPAP and ASV. Other modes may include BiPAP (VPAP), BiPAP S/T, AVAPS, and/or other volume and/or pressure control modalities.

The memory 126 may be any type of device for storing application data as well as other data related to the described operation such as application data, data generated by the system, operating parameters, SSI, SMI, etc., and/or combinations thereof. The application data, data generated by the system, operating parameters, SSI, SMI, etc., and/or combinations thereof, may be received by the controller 120 for configuring (e.g., programming) the controller 120 to perform operation acts in accordance with embodiments of the present system. The controller 120 so configured becomes a special purpose machine particularly suited for performing in accordance with embodiments of the present system.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device or memory, such as the memory 126 or other memory coupled to the controller 120.

The program and/or program portions contained in the memory 126 may configure the controller 120 to implement the methods, operational acts, and functions disclosed herein. The memories may be distributed, for example between the clients and/or servers, or local, and the controller 120, where additional processors may be provided, may also be distributed or may be singular. The memories may be implemented as electrical, magnetic, or optical memory, or any combination of these or other types of storage devices. Moreover, the term "memory" should be construed broadly enough to encompass any information able to be read from or written to an address in an addressable space accessible by the µP 130 of the controller 120. With this definition, information accessible through a network such as the network 108 is still within the memory, for instance, because the µP 130 may retrieve the information from the network 108 for operation in accordance with embodiments of the present system.

The sensor 114 may be situated at one or more portions of the system and may sense related parameters, form corresponding sensor information, and provide this sensor information to the controller 120 and/or to its own controller for further processing. For example, the sensor 114 may include sensors which may be specific to a test type being performed. The sensor 114 may be distributed throughout the system 100 and may communicate with each other and/or other portions of the system such as the controller 120, the TX/RX 128, etc., using any suitable wired, wireless, and/or optical communication method or methods. It is also envisioned that in some embodiments one or more of the sensor 114 may provide sensor information continuously, in response to a query, and/or on at periodic and/or non-periodic intervals. For example, in some embodiments, one or more of the sensor 114 may push data at periodic and/or non-periodic intervals to the controller 120, depending upon system settings as may be set forth in the SSI. In yet other embodiments, it is envisioned that one or more of the sensor 114 may provide data to the controller 120 in response to a query. In some embodiments, one or more of the sensor 114 may transmit bursts of sensor information to the controller 120 at periodic intervals (e.g., every 5 minutes, etc.) to save power by transmitting in burst at periodic intervals rather than transmitting continuously, the latter of which may drain power from a power source especially when operating as a remote wireless sensor 114 such as a remote biosensor.

The controller 120 may be configured to communicate with the MS 104 using any suitable communication method or methods via, for example, the network 108. The controller 120 may obtain the SSI from the MS 104 and store this information in the memory 126 for reference at a later time and/or to configure its operation in accordance with the SSI. During operation, the controller 120 may receive analog signals from the sensor 114, amplify (e.g., using one or more amplifiers) and/or condition these analog signals (e.g., using one or more signal conditioners), convert these signals (e.g., using one or more analog-to-digital (A/D) converters) to digital signals (e.g., at a desired sampling rate (as may be set in the SSI)) to form corresponding information which may be further processed or may be stored in the SMI for later use and/or processing. In some embodiments, it is envisioned that one or more of the sensor 114 may process signals as discussed above, such as via one or more sensor controllers of the one or more of the sensor 114 and transmit corresponding digital signals to the controller 120 for further processing. It is further envisioned that in some embodiments, one or more of the sensor 114 may be wirelessly paired to the controller 120. It is also envisioned that one or more of the sensor 114 may be remotely located relative to the controller 120 of the PAP 102. Accordingly, the remote sensor 114 may communicate with the controller 120 via the Tx/Rx 128 using any suitable wireless communication method or methods which, without limitation, may include Bluetooth^{™},WiFi^{™}, etc.

The PAM 144 may include one or more an air pump(s), blowers, a pressure regulator(s), a flow regulator(s), flow valves, and flow monitor(s) and may be configured to provide airflow at a desired pressure and/or flow (e.g., volume) to the patient under the control of the controller 120. The PAM 144 may be coupled to the patient via the patient interface (PI) 146. The PAM 144 may provide an output in accordance with one or more operational modes, such as a multi-modal continuous positive airway pressure (CPAP) mode or Automatic/Adaptive Servo Ventilation (ASV) mode, under the control of the controller 120.

The patient interface (PI) 146 may include any suitable patient interface that may deliver a flow of air to the patient such as a mask, a nasal canula, and/or the like. The patient interface (PI) 146 may be coupled to the PAM 144 via a line-set and may include one or more sensor 114 of the sensors.

The controller 120 may monitor the sensors 114 and process data to enable pressure management decisions in accordance with embodiments of the present system that may ensure that cardiac output is preserved and/or excessive sympathetic activation is not generated. The controller 120 may analyze sensor information to detect sympathetic activation and may be configured to realize these pressure management decisions by controlling the PAM 144 to be configured to provide flow according to a desired operational mode such as a continuous positive airway pressure (CPAP) mode or an Automatic/Adaptive Servo Ventilation (ASV) mode as may be desired. In ASV mode, positive pressure may be provided when apneas or hypopneas are detected. Detection may be performed in many suitable ways including those disclose by U.S. Patent No. 6,752,151 entitled "Method And Apparatus For Providing Variable Positive Airway Pressure", issued June 22, 2004. However, other modes are also envisioned. The controller 114 is illustratively described as performing analyzing, etc., to simplify the discussion herein. However, in accordance with embodiments, one or more other controllers may perform this operation. Accordingly, the discussion herein regarding the controller 120 should be understood to similarly apply to any one or more of these other controllers.

The sensor 114 may be utilized to aid in cardiovascular characterization by the controller 120 to aid in automated control actions for therapeutic devices, such as the PAP 102. For example, electrocardiogram (ECG) characterization by the sensor 114 of cardiological phenomena may be utilized by the controller 120 to determine cardia-time-intervals. In accordance with embodiments, these time intervals can be used to derive specific analytical characterizations of heart function such as the pre-ejection period, such as when measured noninvasively with ballistocardiogram (BCG) or impedance cardiography by the sensor 114. Combining this periodic description with a continuous, time-based series (e.g., left ventricular ejection time) may be utilized by the controller 120 to derive the contractility coefficient. Insights that are gained from the contractility coefficient include health classifications for sympathetic nervous system function and sodium potassium pump activity. Overall understandings ascertained by the present system from ECG characterization include stroke volume and heart rate. Given variations in these two characteristics, the present system may detect arrhythmias. An arrhythmic heart rate is an indication of diminished cardiac output due to non-uniform blood transport and in response to detection, the controller 120 may control the PAP 102 accordingly as described herein.

In accordance with embodiments, the controller 120 may be utilized via the sensor 114 to characterize pulmonological hemodynamic behavior while on therapy. Specifically, characterization of mean pulmonary arterial pressure (MPAP) is enabled thereby, providing for characterization of CHF specific behavior as diminished cardiac output can be indicated by increased mean pulmonary arterial pressure (MPAP). By using electrical impedance tomography (EIT) provided by the sensor 114, an impedance map of the lungs may be determined by the controller 120. Through time series analysis of the change in skin impedance, pulmonary characteristics can be derived, specifically pulmonary arterial pressure as well as heart rate. These metrics provide for the monitoring of cardiac performance by the system 100. Given how pulmonary artery pressure is a bio-transport phenomenon which measures blood flow into the lungs for filtering, stroke output of the heart is key to function. Combining these metrics allows for a granular measurement of the heart by the controller 120 to mitigate negative effects of congestive heart failure when controlling the PAP 102. Additional sensors may provide confidence in control action, such as a photoplethysmography (PPG) sensor (e.g., sensor 114).

Photoplethysmography (PPG) sensors (e.g., sensor 114) are reflexive sensors which use infrared light to measure transmission or reflection of light in response to left ventricular pressure increases in the heart. These pressure increases sensed by the sensor 114 manifest due to the contraction of the heart. As bio-transport of blood initiates, the volumetric change can be measured by the sensor 114. Such sensory output may be utilized by the controller 120 to derive heart rate, where the volumetric changes provide a measure of the heart rate. In accordance with embodiments, detected variation in heart rate may be utilized as an indicator for arrythmia which denotes diminished cardiac output, which may be utilized to result in suitable control of the PAP 102 as described further herein.

One or more of the sensor 114 may include sensors of one or more types such as pressure sensors, flow sensors, temperature sensors, carbon dioxide sensors (CO₂), optical sensors, electrical impedance tomography (EIT) sensors, electrocardiogram (ECG) sensors, photoplethysmogram (PPG) sensors, galvanic skin response (GSR) sensors, balistocardiogram (BCG) sensors, and/or the like that may be paired to the controller 120 via wired, wireless, and/or optical communication methods and may provide corresponding sensor information thereto. For example, in accordance with embodiments, one or more of the sensor 114 may be wirelessly paired to the controller 120 and may provide sensor information to the controller 120.

In some embodiments, the controller 120 may employ a multi-night algorithm described herein to monitor the patient's risk factors for poor clinical outcomes and make titration decisions to optimize overall patient outcomes by controlling the PAP 102 accordingly to provide the proper pressure and flow to the patient coupled to PAP 102 via the PI 146. This may provide for proper pressure management decisions that may ensure that cardiac output is preserved and excessive sympathetic activation is not generated in the patient. In accordance with some embodiments, the sensory modality of an external sensor (e.g., a remote biosensor) is an EIT sensor, which can provide sensory information that may enable the controller 120 to noninvasively monitor cardiac and pulmonary phenomena and control the PAM 146 accordingly to output an airflow at a desired pressure and flow.

The PAP 102 may include any suitable measurement system or systems (e.g., the sensor 114) that monitor the patient's physiological parameters as they change over time during a study period using one or more test types to determine changes in physiological parameters of the patient from which sleep features of the patient may be derived. These test types may have process flows and operations that may be stored in the SSI and may be selected from the SSI to be set autonomously by the controller 120 without any user intervention, and/or manually by the patient, and/or by the clinician as may be desired. In accordance with embodiments of the present system, it is envisioned that the PAP 102 may, using the sensor 114, monitor the patient's physiology over a study period in accordance with one or more test types (e.g., each corresponding to a different type of study such as full diagnostic polysomnography, an at home sleep study (AHSS), etc.), form corresponding sensor information, process the sensor information in accordance with the corresponding test type, and thereafter store the results in the SMI. The study period may include a time period during which the patient is monitored. This time period may vary depending upon the type of sleep study and information of such may be provided to a controller of the system. The study period may include epochs which, in the present embodiments, may be set to 15 days, although other study periods and/or epoch intervals are envisioned.

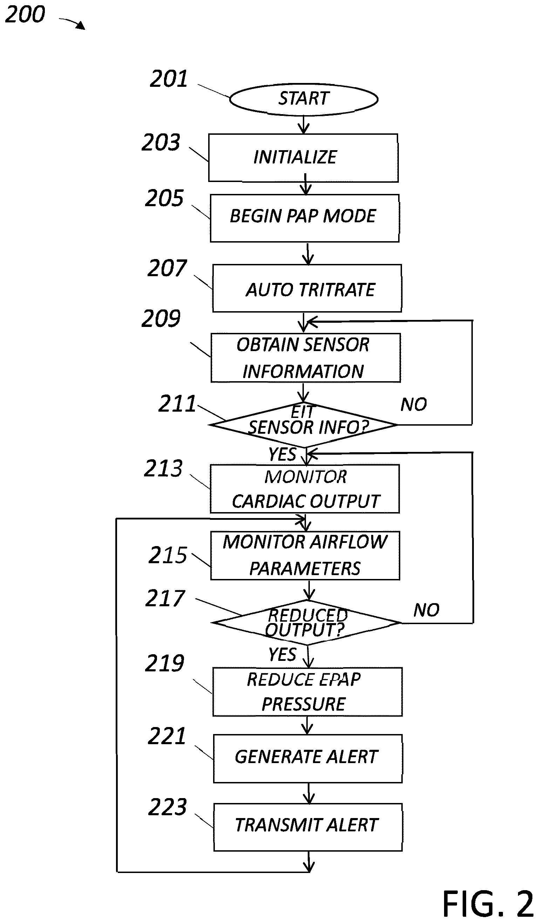

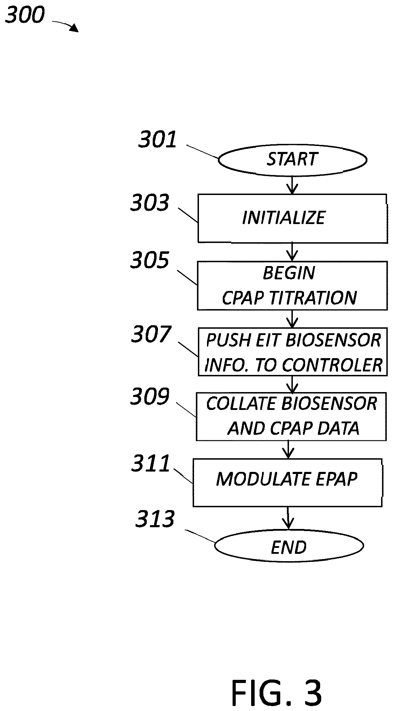

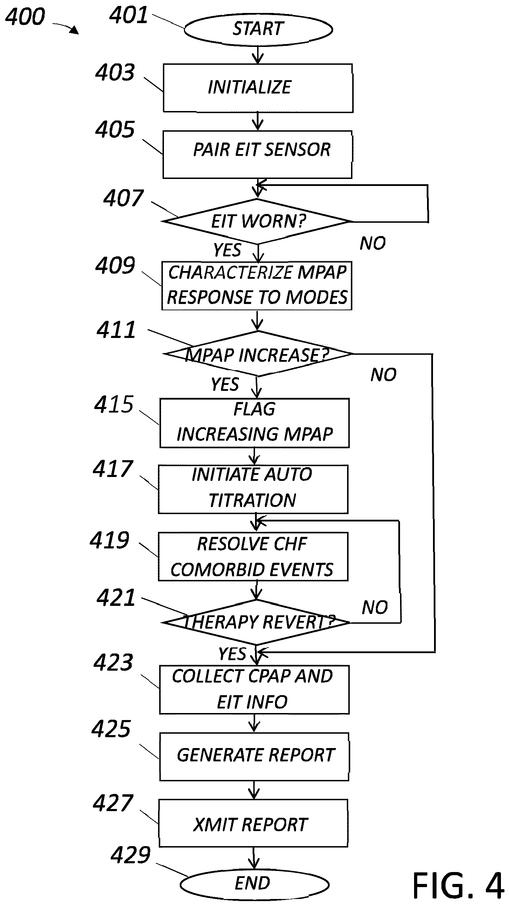

Operational processes performed by the system will now be described with reference to FIGs. 2 through 4 wherein: FIG. 2 illustratively shows a functional flow diagram performed by a process 200 performed for a CHF comorbid patient with SDB patient in accordance with embodiments of the present system; FIG. 3 illustratively shows a functional flow diagram performed by a process 300 for biosensor CPAP data migration process in accordance with embodiments of the present system; and FIG. 4 illustratively shows a functional flow diagram performed by a process 400 for resolving diminished cardiac output in response to ASV therapy with SDB patients comorbid with CHF in accordance with embodiments of the present system.

The processes 200, 300, 400 may be performed using one or more processors, computers, controllers, etc., communicating over a network and may obtain information from, and/or store information to, one or more memories which may be local and/or remote from each other. Accordingly, as referenced herein, such as "the process may ..." refers generally to one or more controllers performing an operation in accordance with embodiments of the present system, though in instances may refer to the operating process (e.g., "the process may start ..."). The processes 200, 300, 400 may include one of more of the following acts. In accordance with embodiments of the present system, the acts of the processes 200, 300, 400 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single PAP and a single patient case. However, without limitation, it should be understood that the process may employ a plurality of PAPs and a plurality of patient cases each of which may include a separate workflow such as a sub-workflow.

With reference to FIG. 2, in operation, the process 200 may start during act 201 and then proceed to act 203 where it may be initialized by performing an initialization routine. During initialization, the process may load SSI and may set system settings in accordance with the SSI. The SSI may further include information related to test type (e.g., study type, e.g., AHSS), the duration of study period (e.g., 15, days, 30 days, etc. although other periods are also envisioned), epochs (e.g., 15 days, although other epochs also envisioned), operation modes (e.g., therapeutic modes), initial operation mode, test process flow, test sensor parameters, and/or other settings. The operation modes may include ASV and/or AutoCPAP modes having PAP control parameters (e.g., EPAPmax, PSmax, Pmax). The PAP control parameters may be defined in the SSI and may be modified by the system and stored as for later use. During the initialization, an EIT sensor, which may be external sensor, may be paired to the controller (e.g., controller 120), automatically, or by the clinician and/or the patient. Accordingly, during operation, the external biosensor such as the EIT sensor may communicate with the controller via a wireless communication link such as Bluetooth^{™},WiFi^{™}, and/or the like. The process may also begin an application interface (API) configured to enable wireless communication between the controller and one or more sensors of the system such as an external biosensor (e.g., the EIT sensor, etc.). After completing act 203, the process may continue to act 205.

During act 205, the process may begin a positive airway pressure (PAP) operational mode (e.g., a PAP mode). For example, the process may start in AutoCPAP mode, and the sensors may monitor a key parameter such as CPAP pressure. PAP pressure would titrate to resolve sleep-disordered breathing events (e.g., apnea, hypopnea, snoring). A detected decrease in cardiac output and/or increase in patient sympathetic output associated with the pressure increase by the PAP device, In response to such change, a limit may be set to an AutoCPAP pressure to below the pressure at which the decrease in cardiac output or increase in sympathetic activation is detected. In accordance with further embodiments, the device may start in ASV mode, and the sensors may monitor a key parameter such as EPAP. PAP pressure (specifically EPAP) titrates for SDB events. In response to a detected decrease, a limit may be set to a Limit EPAPmax setting (i.e., don't allow EPAP increases to reduce cardiac output).

The PAP mode includes control parameters as may be set forth by the SSI. Accordingly, the system may begin to monitor the patient and may supply positive airway pressure to the patient depending upon settings. For example, it should be appreciated that in some embodiments, the positive airway pressure or PAP mode may only monitor airflow to, or from, the patient during a titration period for 15 days before any airway pressure is provided, for example as may be set forth in the SSI. In some embodiments, the PAP operational mode may start at a baseline amount of pressure in order to flush CO2 from the system. In this way a minimal CPAP or EPAP pressure is applied. In this way, there may be no pressure support (i.e., no additional support for inhalation). In other words, no therapeutic (air) pressure is applied. This may assure that before applying therapeutic pressures, homeostatic trends may be established to determine if cardiac output has diminished significantly. Naturally, one or more predetermined baseline level(s) of support may be defined, for example, as a portion of the SSI. In an alternative embodiment, no PAP may be applied at all to generate baseline cardiac output and sympathetic activation parameters. For example, by starting assessing baseline measurements prior to applying any PAP pressure, then a baseline measurement (or series of measurements) is determined to compare to each other. These vary throughout the night, so statistical means may be utilized to determine if (and at what pressure) the therapeutic gas support modifies cardiac output by a statistically (and clinically) significant amount. A similar statistical approach may be utilized when some baseline level of PAP support is provided. For example, cardiac output may be monitored for several days, prior to applying PAP therapy to see if a significant decrease occurs with the PAP therapy.

Further, in order to baseline control parameters such as expiratory positive airway pressure (EPAP), a response function of mean pulmonary arterial pressure (MPAP) with respect to EPAP may be created and stored in a memory of the system for later use. The baseline control parameters may then be stored in the system memory 126 as the initial system settings included as a portion of the SSI for the particular patient or subject under treatment. It is also envisioned that the process may, depending upon settings, sense cardiac output trending for use as an example function, where the cardiac output function may be represented as the response function or map of MPAP versus EPAP, for example. Accordingly, the system may compare a previous value of cardiac output such as the MPAP (e.g., obtained from sensor information at a previous setpoint, e.g., 15 minutes, most recent, etc., as may be set in the SSI) with a current value of cardiac output. Accordingly, if it is determined that the previous value for cardiac output is greater than or equal to the current value of cardiac output, it may be determined that cardiac output has not decreased. However, if it is determined that the previous value for cardiac output is less than the current value of cardiac output, it may be determined that cardiac output has decreased, e.g., as indicated by an increase in MPAP (i.e., trending that patient cardiac output has diminished (e.g., MPAP has increased) to a significant extent). Trending of the cardiac output parameter (e.g., MPAP) may be mapped as a function of a control parameter (e.g., EPAP pressure) of the PAP machine. The system may identify/stratify pressure sensitive vs. pressure insensitive patients this way. Thus, the pressure sensitivity of the patient may be identified. For example, the processor 130 may determine or be configured to determine that a particular patient is pressure sensitive when the patient's cardiac output changes by a predetermined cardiac amount in response to a predetermined EPAP pressure change. The predetermined cardiac amount and the predetermined EPAP pressure change may be experimentally, empirically and/or statistically determined based on data from the particular patient and/or based on average data from a group or population of patients similarly situated as the current patients. As appreciated, even for a given patient, their status may change over time. As such, in accordance with the present system, a particular patient may be re-baselined periodically. In accordance with embodiments, the re-baseline may be either at minimal PAP therapy and/or without PAP therapy. In this way, the PAP therapy may be adapted to individuals' specific responsiveness to PAP (i.e., how much their cardiac output or sympathetic activation is impacted by increased pressure).

After completing act 205, the process may continue to act 207 during which the process may begin a titration period in which it may titrate automatically using the additional information supplied by the external biosensor such as the EIT sensor. This titration period may have settings and may last for a period of time as may be set forth in the SSI and/or may be set by a clinician. For example, in the present embodiments, it is envisioned that the titration period is 30 days although other periods of time are also envisioned such as a two-week or two-month period. In accordance with some embodiments, it is envisioned that the titration period may be divided into 15-day epochs (although epochs of other lengths are envisioned) where monitoring for the need to initiate ASV or PAP mode (such as an AutoCPAP mode) may be performed. Accordingly, each mode may receive 15 days of data collection and surveillance. In some embodiments, a period of baseline data may also be captured (e.g., for 15 days prior to PAP initiation) as may be set forth in the SSI. The epochs for each mode (e.g., ASV, AutoCPAP, AutoPAP, which are effectively synonymous in the art while AutoCPAP may be considered slightly more specific, in that it means that the inspiratory and expiratory pressure are effectively the same and AutoPAP might also include Auto BiPAP, etc.) or for baseline data may be set forth in the SSI and set by the system and/or clinician. After completing act 207, the process may continue to act 209.

During at 209, the process may obtain sensor information from one or more sensors of the system (e.g., the sensor 114), such as from the external biosensor including the EIT sensor. Accordingly, the controller may monitor the sensors to receive information therefrom. The external biosensor, such as the EIT sensor, may automatically begin to transmit (e.g., push) sensor information when the external biosensor(s) detects that it is attached to a patient. It is envisioned that the external biosensor may, for example, push sensor information to the controller or may provide sensor information upon a request from the controller (e.g., a query, etc.). In some embodiments the external biosensor may include a sensor controller/processor (which may be part of and/or separate the system controller 120) that analyzes the sensor information and determines whether a cardiac event is detected and, if so, may push sensor information indicative of this to the controller. This information may include, for example, a flag indicating that a cardiac event occurred. Accordingly, the external biosensor may transmit when it detects that a cardiac event has occurred or may transmit data in bursts at periodic or non-periodic intervals or a combination of periodic and non-periodic intervals. This may conserve battery life of an onboard power supply of the external biosensor. Although a single external biosensor is discussed, it is envisioned that there may be more than one external biosensor. After completing act 209, the process may continue to act 211.

During act 211, the process may determine whether sensor information from the external biosensor, such as the EIT, was detected and transmitted by the external biosensor and received by the controller 120 and/or other devices and controllers connected to the system through a wired and/or wireless connection such as via the network 108, for example. Accordingly, when it is determined that sensor information from the EIT was received, the process may continue to act 213. However, when it is determined that sensor information from the EIT was not received, the process may repeat act 209. The EIT may transmit sensor information to the controller as a result of a request to do so (e.g., a query) or may push information to the controller at periodic or non-periodic intervals, for example, in response to detection that the EIT makes contact with the skin of the subject/patient and/or in response to detection that a cardiac event occurred and/or has otherwise been detected.

During act 213, the process may monitor the cardiac output of the patient. Accordingly, the process may receive information from one or more of the external sensors, such as from one or more of the external biosensors such as from the EIT, an ECG, a PPG, a coupled BCC and/or an ECG sensor, etc., which may provide information indicative of cardiac output. For example, PPG/ECG may monitor for sympathetic activation. EIT for monitoring Cardiac output may be suitably applied such as by incorporating teachings such as provided by: Proença, M., Braun, F., Lemay, M., Solà, J., Adler, A., Riedel, T., ... & Rexhaj, E. (2020), entitled "Non-invasive pulmonary artery pressure estimation by electrical impedance tomography in a controlled hypoxemia study in healthy subjects." Scientific reports, 10(1), 1-8. After completing act 213, the process may continue to act 215.

During act 215, the process may monitor the airflow (e.g., patient airflow) parameters to maintain stable respiration through pressure control of components of the PAP device 102, such as the PAM 114, for example. Accordingly, a controller of the system may obtain sensor information from the sensors of the system such as the airflow sensor and/or the pressure sensors to obtain flow and pressure information, respectively, which information may then be compared to airflow/pressure threshold settings as may be set in the SSI. The controller may then be operative to match the airflow and/or pressure settings with that set forth in the SSI and may control the PAM accordingly to be operative in accordance with set airflow and pressure parameters as may be defined by a current operating mode (e.g., PAP mode, etc.).

After completing act 215, the process may continue to act 217 during which the process may determine whether there are indications of reduced cardiac output (i.e., an aspect of cardiac output has decreased). Accordingly, when it is determined that there are indications of reduced cardiac output, the process may continue to act 219. However, when it is determined that there are no indications of reduced cardiac output, the process may repeat act 213. There are several methods that may be employed by embodiments of the present system to determine whether there are indications of reduced cardiac output, one or more of which may be utilized in accordance with embodiments of the present system. While exemplary methods are discussed herein, it is appreciated that other methods may also be suitably utilized. When any of these are determined in the affirmative, the process may determine that there is reduced cardiac output.

In accordance with a first exemplary method, it is envisioned that the system may monitor information from the external biosensor such as the EIT sensor to determine MPAP. Accordingly, when it is determined that the MPAP is equal to or greater than 25 mmHg, the process may determine that cardiac output is reduced (e.g., cardiac output has diminished). However, when it is determined that the MPAP is less than 25 mmHg, the process may determine that cardiac output is not reduced (e.g., has not diminished). The process may determine that there is reduced cardiac output when one or more conditions, depending upon system settings, may be met. For example, reduced cardiac output may be determined by monitoring a response function of the MPAP with respect to the EPAP. This may establish the pressure sensitivity of the patient. Thus, the process may, in order to determine baseline control parameters such as EPAP, form a response function of MPAP with respect to EPAP. Baseline data may provide a statistical model of cardiac output (with either no PAP pressure or minimal PAP pressure). In this way, during PAP titration, the present system may determine if the cardiac output has significantly decreased as a function of increased PAP pressure. Typically, initial operating parameters may be set at low pressures regardless and are titrated upwards as the patient show ventilatory needs.

This method provides for a description of pulmonological hemodynamic behavior while on therapy. Specifically, characterization of mean pulmonary arterial pressure (MPAP) may be enabled and monitored allowing for characterization of CHF-specific behavior as diminished cardiac output which may be indicated by increased MPAP. By using one or more EIT sensors, an impedance map of the lungs may be created by the system and stored in a memory of the system. As the one or more EIT sensors sense skin impedance of the subject, the process may then, through time series analysis of the change in skin impedance, derive pulmonary characteristics. Specifically, pulmonary arterial pressure as well as heart rate may be derived from sensor information received from the one or more EIT sensor. These metrics allow and enable monitoring of cardiac performance. Given how pulmonary artery pressure is a bio-transport phenomenon which measures blood flow into the lungs for filtering, stroke output of the heart is key to function (e.g., of primary significance). Coupling these metrics allows for granular measurement of the heart to mitigate negative effects of congestive heart failure.

In a second exemplary method, the process may determine whether there is reduced cardiac output when an arrhythmic heart rate is detected. Accordingly, when it is determined that an arrhythmic heart rate is detected, the process may determine that there is reduced cardiac output. However, when it is determined that an arrhythmic heart rate is not detected, the process may (absent other contrary determinations) determine that there is no reduced cardiac output. Generally, an arrhythmic heart rate is an indication of diminished cardiac output due to non-uniform blood transport. The presence of an arrhythmia may be detected using any suitable method such as through analysis of ECG sensor information that may be transmitted to the controller. When variability in heart rate is detected, it may be determined that arrythmia is detected as described by a time series deviation in impedance. The external biosensor may include the ECG sensor(s) which may form the ECG sensor information.

During act 219 (which is responsive to the determination in act 217 that there are indications of reduced cardiac output), the process may change system parameters, such as reduce expiratory positive airway pressure (EPAP). Accordingly, the controller may be operative to control the PAM to reduce expiratory positive airway pressure (EPAP) by an amount as set forth by a pressure threshold value (e.g., 1 cm of H₂0 ) every threshold time period (e.g., 20 minutes, although other times are also envisioned) as may be set forth in the SSI. Accordingly, the EPAP pressure on the PAP may be reduced by 1 cm H₂0 each time (e.g., every 20 minutes up till a minimum predetermined/preset EPAP pressure is reached, for example, and/or resolution of the existing issue such as achievement and resumption of normal cardiac output), the process requests that the EPAP be reduced. The process may measure EPAP pressure continuously and may change the EPAP as necessary. For example, the controller may be operative such that EPAPmax will decrement 1 cm of H₂O every 20 minutes, for example, until resolution of the cardiopulmonary insult (e.g., cardiopulmonary event), as classified by the EIT. This approach allows for ventilatory titration mediated by cardiac and pulmonological means. Accordingly, PAP control parameters (e.g., EPAPmax, PSmax, Pmax) may be modified if cardiopulmonary parameters indicate a reduced cardiac output or decreased high-frequency heart rate variability (HF HRV). PSmax is the maximum pressure support (IPAP-EPAP) that is considered provided by the present system. In accordance with the present system, it may be determined to limit the amount of maximum pressure support available from the device such as when it is determined that high pressure support is causing issues with either sympathetic activation or cardiac output. Similarly, Pmax is considered the maximum pressure provided by the device (e.g., may default to 30 cm H2O). In a similar fashion, the present system may determine to limit the overall maximum pressure in addition to or in place of PSmax.

After completing act 219, the process may continue to act 221 during which the process may generate an alert message which may include information indicative of the cardiac event, including extent of the reduced cardiac output and time and date thereof, such as "Reduced Cardiac Output detected 1:04 am, Tuesday September 20, 2022". Illustratively, the extent of the reduced cardiac output may be provided as being continuously rendered or being rendered in response to requesting such additional information. For example, the "Reduced Cardiac Output" message alert may be displayed with an indication that further information is available, such as being highlighted, where, for example, the additional information may be displayed when the cursor is placed on this highlighted "Reduced Cardiac Output" message, such as providing the level of the increased/decreased PAP for example. The alert message may then be transmitted during act 223 using any suitable method to a desired clinician (e.g., the doctor of the patient) for further review and analysis. Accordingly, the process may obtain a contact address of a clinician that should receive this message such as an email address, a text number, a telephone number, a work address, etc., and may transmit the alert to this contact address during act 223. There may be one or more contact addresses to which the alert may be transmitted and these addresses may be stored in a memory of the system such as in the SSI. Accordingly, the system may access SSI to obtain the contact address information. After completing act 223, the process may repeat act 215. It should be noted that although acts 221, 223 are illustratively shown to follow completion of act 219, these acts may also or in place of, occur following act 217 and/or during act 219.

With reference to FIG. 3, in operation, the process 300 may start during act 301 and then proceed to act 303. During act 303, the process may be initialized by performing an initialization routine. During initialization, the process may load SSI and may set system settings in accordance with the SSI, such as dependent on initial mode selection. For example, if AutoCPAP, then AutoMin = 4 cm H2O, AutoMax = 20 cm H2O. If ASV, EPAPmin = 4 cm H2O, EPAPmax = 25 cm H2O, PSmin = 0 cm H2O, PSmax = 21 cm H2O, Pmax = 30 cm H2O, Backup rate = Auto. During the initialization, at least one sensor such as an EIT sensor, which may be an external biosensor (hereinafter both of which may be referred to as EIT sensor for the sake of clarity), e.g., external to the PAP 102, may be paired to the controller, automatically, or by the clinician and/or the patient. Further, pairing may be performed previously, such as when the system is given/assigned to the patient, in which case, the EIT sensor is simply connected to the controller during act 303. Accordingly, during operation, the EIT sensor may communicate with the controller via a wired and/or a wireless communication link such as Bluetooth^{™}, WiFi^{™} and/or the like.

The process may also begin the application interface (API) configured to enable communication with one or more sensors of the system such as the external biosensor (e.g., the EIT sensor, etc.). The system may initialize, for example, for home titration for congestive heart failure patients using a multi-modal sensory approach of a PAP device paired with the EIT sensor. The EIT sensor may be the modality of choice as it enables noninvasive monitoring of cardiac and pulmonary phenomena, though other modalities are contemplated in accordance with the present system. Specifically, this illustrative sensory method characterizes pulmonary measures, such as pulmonary arterial pressure, and cardiac performance measures, such as variabilities in heart rate indicative of arrythmia, as may, for example, be detected through a time series deviations in impedance. PAP control parameters (e.g., EPAPmax, PSmax, Pmax) may be modified when cardiopulmonary parameters indicate a reduced cardiac output and/or increased/decreased HRV, for example due to HF. Before titration begins, the EIT sensor may be configured by a clinician to pair with a PAP device of the patient as discussed. This wireless communication may be enabled using any suitable wireless communication methods such as Wi-Fi^{™}, Bluetooth^{™},etc. Both the external biosensors (e.g., the EIT sensor) and PAP may then be used in a home environment by the patient. During use, the patient will attach the EIT sensor to the patient's body as well as attach the PAP mask before going to sleep.

In response to automatically detecting the attachment (e.g., by the sensor and/or controller of the sensor and/or the controller of the system, such as detecting contact with the skin of the patient/subject), the EIT sensor may begin transmitting (e.g., immediately, periodically, non-periodically, etc., as discussed) sensor information to the PAP device. Upon sensing the transmission of sensor information from the EIT sensor, the PAP device and the EIT sensor may then automatically begin communication via the API such that there is no need for any input required from the patient, clinician, etc. Accordingly, the EIT sensor may include a controller which may detect that it is coupled to (e.g., worn by) the patient and may thereafter transmit sensor information to the controller of the PAP device automatically without user input.

After completing act 303, the process may continue to act 305 during which the process may begin a PAP titration operational mode of the patient, such as an SDB patient with CHF, though other patient ailments (e.g., chronic ailments, such as COPD, etc.) are considered as part of the present system. It is envisioned that the present system may also be used for opioid-induced CSA or other ventilation modes (e.g., neuromuscular, etc.). Accordingly, the process may control the PAM to supply a flow of air to the patient accordingly, as may be set forth in the SSI (e.g., as determined during an initial epoch). After completing act 305, the process may continue to act 307 during which the EIT sensor may push sensor information to the controller of the PAP. Thus, as the titration period is underway, the PAP may occasionally pull sensor information from one or more external biosensors such as the EIT (e.g., via the API which may enable wireless communication), or receive pushed sensor information (e.g., via the API which may enable wireless communication between the EIT and the controller of the CPAP), from the external biosensors, such as the EIT sensor, as well as from other sensors of the system.

After completing act 307, the process may continue to act 309 during which the process may collate the sensor information received from the external biosensor such as the EIT, with sensor information from sensors of the CPAP that has been collected (e.g., CPAP sensor information such as IPAP, EPAP, Breath Rate, Heart rate variability, heart rate, leak, etc., for example, collected by Flow sensors in the PAP machine. , collected by Patient pressure is in accordance with embodiments, considered a key parameter (our control output), as will be reported patient events (e.g., apneas, hypopneas, etc., which may be utilize as the control inputs for the PAP device control input) , for example). As respiratory and cardiac analysis is collated, out of bounds thresholds such as 3 or 4 percent oxygen desaturation, as indicated, for example, by peripheral capillary oxygen saturation (SpO₂) , may be determined. As a diagnostic criterion for OSA, 3 or 4 percent desaturations in a truncated period of time is indicative of OSA disease state in accordance with embodiments. These measures may be used in order to make PAP titration decisions (e.g., increasing pressure in response to O² desaturation events). These determined thresholds may be things such as low or out-of-range SpO₂, MPAP, etc. which may be compared with corresponding threshold values to determine whether they are high/low and/or out of range. In the affirmative where measured values are high/low and/or out-of-range, the process may generate an indication of such and may transmit such information, for example, as alerts to a predetermined recipient such as a medical professional.

In addition or alternately, measured/sensed information, indications and/or alerts may be stored for later access, such as for further review and analysis in the future. In another embodiment, the CPAP may push, for example, automatically in response to a change/high/low and/or out of range measurement/determination, collated data up to a cloud-based server, such as the CSC 106, which may then analyze the information provided thereto to determine whether SpO₂, MPAP, etc. are changed/high/low and/or out of range and may generate an indication of such which may then be transmitted to a clinician for further review and/or may otherwise generate an alert as discussed (e.g., see, FIG. 2, acts 221, 223). Thus, the cloud platform may interpret the data and provide a full report available to the physician for analysis/prescription.

After completing act 309, the process may continue to act 311 during which the process may modulate EPAP pressure, for example, based on an ideal MPAP which may, for example, be predetermined such as experimentally from historical data of the particular patient/subject using the PAP device 102 and/or statistically from average values of a population of similarly situated patients. Accordingly, when indicated by a sensor measurement (e.g., change/high/low and/or out of range measurement/determination), the process may be operative to repeatedly (e.g., every threshold time period such as 20 minutes, etc.) adjust (e.g., increase/decrease) pressure on the EPAP by a threshold value (e.g., by 1 cm of H₂0) every 20 minutes (e.g., a threshold time period) until MPAP has returned (e.g., decreased) to normal levels such as 25 mmHg. After completing act 311, the process may continue to act 313 where it may end.

With reference to FIG. 4, in operation, the process 400 may start during act 401 and then proceed to act 403. During act 403, the process may be initialized by performing an initialization routine. During initialization, the process may load SSI and may set system settings in accordance with the SSI, such as the initial control parameters discussed herein, such as in connection with act 303. During the initialization, at least one sensor such as an EIT sensor, which may be an external biosensor, may be paired to the controller, automatically, or by the clinician and/or the patient. Accordingly, during operation, the external biosensor, such as the EIT sensor, may communicate with the controller via a wired and/or wireless communication link such as a Bluetooth^{™}, WiFi^{™} and/or the other communication links. The process may also begin the application interface (API) configured to enable communication with one or more sensors of the system such as the external biosensor (e.g., the EIT sensor, etc.). This act may be similar to acts 203, and 303, accordingly, reference is made to the description of acts 203, and 303.

After completing act 403, the process may continue to act 405 during which the process may pair the external sensor, such as an EIT sensor, in the present illustrative embodiment, to a controller of a PAP, such as a CPAP, so as to enable wireless communication between the external sensor and the controller of the CPAP. For simplicity, a CAPA is illustratively described, however, in accordance with embodiments, other PAP devices may be suitably utilized. Accordingly, the process may enable the API that may enable wireless communication between the external sensor and the controller of the CPAP.

After completing act 405, the process may continue to act 407 during which the process may determine whether the EIT sensor (e.g., the external biosensor) is worn by the patient which may be indicated by the user or determined automatically without user input (e.g., by the sensor and/or controller of the sensor and/or the controller of the system) as discussed herein such as in connection with act 303. Accordingly, when it is determined that the EIT sensor is worn by the patient, the process may continue to act 409. However, when it is determined that the EIT sensor is not worn by the patient, the process may repeat act 407. The process may determine that the EIT sensor is worn by the patient, for example, when it is sensed that the external EIT sensor begins transmitting sensor information to the controller of the CPAP. This may be done via a wireless communication link (e.g., enabled by the API) and may occur automatically when the EIT sensor senses physiological parameters of the patient which is an indication of being coupled to the patient, such as in contact with the skin of the patient. As appreciated, other systems of sensing that the sensor is worn by the patient may in addition or in place of the controller sensing/receiving sensor information may be suitably applied. For example, the sensor may utilize an ohmic measurement to detect contact with skin of the patient and transmit an indication of such to the controller. In any event, the EIT sensor may push sensor information to the controller and/or may transmit an indication that it is contact with the skin of the patient (e.g., automatically without user intervention) at periodic and/or non-periodic intervals and/or may transmit sensor information in response to a request from the controller and/or in response to a physiological signal (e.g., sensed physiological signal when compared with corresponding threshold values is determined high/low and/or out of range).

During act 409, the process may characterize MPAP response to one or more CPAP modes. For example, the paired sensor (e.g., the EIT sensor), as well as one or more other sensors, may provide sensor information that may be processed to characterize MPAP response to the EPAP during CPAP modes, for example, by monitoring the MPAP in relation to the EPAP and CPAP modes. For example, the MPAP may be mapped as a function of EPAP and CPAP. The process may further monitor the EIT sensor and/or the one or more other sensors information for arrythmias, etc.

After completing act 409, the process may continue to act 411 during which the process may determine whether there is an increase in MPAP. Accordingly, when it is determined that there is an increase in MPAP, the process may continue to act 415. However, when it is determined that there is no increase in MPAP, the process may continue to act 423. Accordingly, the process may monitor MPAP over time and may compare a current reading of MPAP with a previous reading (e.g., taken at a threshold delay time Δt = 15 minutes ago, however other values of Δt are also envisioned) of MPAP to determine if the current MPAP has increased (or decreased) relative to the previous reading. In accordance with embodiments, increased MPAP will characterize CHF-specific behavior as diminished cardiac output. Thus, the process may determine there are indications of reduced cardiac output (cardiac output has decreased) by monitoring MPAP and determining that the MPAP increased, for example, by a threshold amount over time, such as an MPAP increase of at least 10% above a normal level (e.g., 25 mm Hg), for example, to 27.5 mm Hg which remains elevated or keeps increasing over 2-3 minutes, for example, or until cardiac output resolves MPAP in the lung. Different time scales for impact may be evaluated (e.g., 2-3 mins as an exemplary minimum), up to 30 minutes may be necessary due to signal-to-noise considerations, for example. This act may be performed at the EIT sensor and/or one or more other sensors and/or may be performed by a controller that is separate from the sensor.

During act 415, the EIT sensor may flag the MPAP increase and transmit corresponding information to the controller of the CPAP device using the wireless communication link established by the API. The flag may be transmitted alone or together with sensor information from the EIT sensor which may indicate that MPAP has increased and, thus, cardiac output has decreased. The flag and/or the sensor information and may, for example, indicate a current time and date, the current sensor readings, etc. The flag may be placed as a leading bit, trailing bit, etc., in accordance with embodiments, Any payload which indicates a disease state may be sent to the physician as a general data package. After completing act 415, the process may continue to act 417 during which the process may initiate auto titration in response to the controller of the CPAP device receiving the information indicative of the flagged MPAP increase/decrease from the EIT sensor. Accordingly, the process may begin auto-titrating CPAP therapy to resolve an increasing MPAP event determined during act 411 and to restore cardiac output to homeostatic levels, such as by decrease EPAP from a maximum EPAPmax which also may be incrementally decreased, for example. So, if the pt MPAP is increasing, then LVEF is decreasing, which means that EPAP is likely too high. So, the PAP titration decision would be to decrease pressure. So, functionally, EPAPmax may be decremented in order to prevent subsequent EPAP increases. The settings of the CPAP device may be titrated on the basis of the airflow characteristic(s), the ventilation characteristic(s) and/or the cardiac characteristic(s). It is envisioned that the controller may control the PAM such that EPAPmax will decrement by a difference in pressure Δp over a threshold time period Δt which may be represented as Δp/Δt until resolution of the cardiopulmonary event and restoration of normal cardiac output. The mode may be AUTOCPAP/AUTOTITRATE MODE OR may be switched to ASV mode. Either of the two options may be suitable. For example, if the device is in an AutoCPAP mode, the mode may be maintained or changed to ASV, etc. In the present embodiments, Δp may be set to 1 cm and Δt may be set to 20 minutes, however, other values are also envisioned and may be set by the system and/or clinician and may be stored in the SSI. Thus, the process may control the PAM such that EPAPmax will decrement 1 cm of H2O every 20 minutes until the cardiopulmonary event is determined to be resolved or until a minimum predetermined EPAPmax pressure is reached, as may be set by the system and/or clinician and may be stored in the SSI. This approach allows for ventilatory titration mediated by cardiac and pulmonological output.

After completing act 417, the process may continue to act 419 during which the process may attempt to resolve CHF comorbid events. In accordance with embodiments, all therapeutic control actions may be mediated by ventilatory and/or cardiac metrics as they pertain to CHF comorbid manifestations of OSA and CSA. The controller of the system may determine the mode it is in and may be operative to control the PAM to automatically revert therapy modes, for example, from ASV to AutoCPAP (where PAP device setting(s) revert to the settings included in the SSI, for example, as determined after the initial titration process in response to the cardiopulmonary event (e.g., see act 417) being resolved.. In accordance with the present system, the process may go from AutoCPAP to ASV mode in response to a noted cardiac events in the titration process. AutoCPAP may also be changed to AutoBiPAP with the intent to keep mean airway pressure lower to avoid impacting cardiac output). Thus, the CPAP may revert therapy modes from ASV to AutoCPAP. In accordance with some embodiments, after an initial 15-day span where observation on needs for ASV therapy has elapsed, if it is determined that there is no longer a need for ASV therapy, the therapeutic mode may automatically change to AutoCPAP mode.

After completing act 419, the process may continue to act 421 during which the process may determine whether the therapy mode has reverted from ASV to AutoCPAP. Accordingly, when it is determined that the therapy mode has reverted from ASV to AutoCPAP, the process may continue to act 423. However, when it is determined that therapy mode has not reverted from ASV to AutoCPAP, the process may repeat act 419. For example, the CPAP may revert based off all clear signal from EIT sensor. The process may perform this because the cardiac event has been resolved, thus dynamic pressure indexing is no longer required, allowing for continuous PAP to be delivered until another cardiac event is detected, for example.

During act 423, the process may collect CPAP and EIT sensor information generated during the process. This may be accomplished, for example in the cloud (e.g., at a remote server), at the CSC 106, etc., such that information generated by the process may be collected at a central location for storage and/or processing. Accordingly, the system may transmit information from the PAP to the CSC, for example, via any suitable network.

After competing act 423, the process may continue to act 425, during which the process may generate a (clinical) report based upon the information sensed, determined, generated, collected, etc., during the process, for example, during one or more epochs (e.g., set to 15 days). Accordingly, the CSC may interpret the collected information generated by the process and may generate the report including a prescription. This report may include preferred therapy settings for the patient. Following another 15 day epoch, a 30-day report may be generated by the system and may include prescription sets and therapy modes. As such, act 425 may be repeated one or more times (e.g., once for an initial setup epoch, and one or more additional times/epochs) The result of this reporting mechanism enables, for example, homeostatic treatment of SDB pertaining to OSA and CSA as influenced by CHF. It is also envisioned that embodiments of the present system may be configured for continuous patient usage, in which case OSA treatment may be optimized such that SDB may also be treated (e.g., optimally), and cardiac output and sympathovagal balance may be preserved.

After completing act 425, the process may continue to act 427 during which the process may transmit the report to one or more clinicians (e.g., the treating doctor, etc.) as may be set forth in the SSI. The transmission may occur via any suitable method such as text message, email, social network, work address, short message service (SMS), etc., as may, for example, be set forth in the SSI. Accordingly, the desired clinician(s) may receive the report and view it at their convenience. After completing act 427, the process may continue to act 429 where it may end.

In accordance with some embodiments of the present system, the process may perform a multi-night protocol for titration beginning with auto-titrating CPAP therapy to resolve apnea/hypopnea events. This may be inclusive of desaturations and arousals, as they will be indicated by the EIT sensor. When it is detected that sleep disordered breathing is sustained, the process may automatically switch a therapy mode to an appropriate mode for central sleep apnea (e.g., ASV therapy). In accordance with embodiment, the settings of the PAP device may be titrated on the basis of both the airflow characteristic(s) ventilation characteristic(s) and/or the cardiac characteristic(s). In accordance with some embodiments, OSA treatment may be optimized such that SDB may be successfully treated while cardiac output and/or sympathovagal balance are preserved.

In accordance with embodiment, therapy modes may also depend on event severity over time. For example, event severity may be described as severe AHI > 15, MPAP > 25% or left ventricular ejection fraction as determined by EIT being much greater than 35%. In some embodiments, after automatically switching from the initial Auto-titrate mode (also referred to as AutoCPAP mode) to ASV mode, an initial 15-day span and/or subsequent span may be utilized to access needs for continued ASV therapy, and when it is determined that there is no longer a need for ASV therapy, for example in response to a sustained period of time > 15 days where EIT indicates MPAP is less than 25 percent, a therapeutic mode may automatically change to or change back to AutoCPAP mode. It is also envisioned that the PAP device may be titrated on the basis of both the airflow/ventilation characteristic(s) and the cardiac characteristics. All therapeutic control actions may be mediated by ventilatory and/or cardiac metrics as they pertain to CHF comorbid manifestations of OSA and CSA. Following another epoch (e.g., a second 15-day epoch), a 30-day report may be generated containing prescription sets and corresponding therapy modes. The result of this reporting mechanism enables homeostatic treatment of SDB pertaining to OSA and CSA as influenced by CHF.

In some CHF patients at some period(s) in time, treatment of periodic Cheyne-Stokes Respiration (CSR) markedly increases sympathetic activation (i.e., in these patients, CSR can serve as a compensatory mechanism for their CHF and thus CSR may not be treated). In accordance with embodiments of the present system that may be targeted toward these patients, sympathetic activation of the patient is trended prior to, and/or during, treatment. In these embodiments, quantifying patient sympathetic nervous system sensitivity before PAP therapy application is imperative. Thus, the system may establish a homeostatic trend before PAP therapy using GSR or HRV methods. This pre-processing may enable baseline sympathetic nervous system activity establishment. In accordance with embodiments, in the case of increased sympathetic (where the system may observe the sensitivity of the patient) nervous system activity (e.g., indicating decreasing therapeutic benefit in the CHF of the patient), the following sensor mediated control action may trigger: If alkalosis and hypokalemia are present, this indicates that sympathetic nervous system activity has exceeded its normal bounds of less than or equal to 30 µS, then PSmax on the CPAP may be reduced by 1 cm of H₂O. To determine whether alkalosis and hypokalemia are present, the process may determine whether galvanic skin response (as measured by the GSR sensor) is greater than 30 µS. Accordingly, when it is determined that the galvanic skin response is greater than 30 µS, then the process may determine that alkalosis and hypokalemia are present. However, when it is determined that the galvanic skin response is less than or equal to 30 µS, the process may determine that alkalosis and hypokalemia are not present. It is also envisioned that the process may determine whether alkalosis and hypokalemia are present using sensors other than the GSR sensor, such as an ECG-based HRV sensors, PPG sensors, BCG sensors, EIT sensors, and/or GSR sensors. Referring back to PSmax, this pressure reduction process may continue until an electrodermal activity has returned to a normal level (≤ 30 µS). It is also envisioned that sympathetic activation may also include pressure support ventilation and/or sympathovagal balance. In accordance with embodiments, the system may determine whether there is increased sympathetic activation as well as determine sensitivity of the patient to a control parameter.

In another embodiment, further control actions may be mediated via ECG and/or PPG characterization of HRV (e.g., LF/HF ratio). Before delivering therapy, a homeostatic trend of the CHF patient may be established, for example, via ECG. Specifically, statistical measures (i.e., mean, and standard deviation (SD) from the mean) on the LF/HF ratio during sleep for a one week period, for example, may be measured. When, during treatment of CSR with the ASV device, nighttime LF/HF ratio is determined to exceed a baseline, for example, + 2 SD LF/HF ratio, then the PSmax of the ASV device may be decreased until a LF/HF ratio decreases to within baseline range as may be set forth by a baseline range threshold. In accordance with embodiments of the present system, the LF/HR ratio of the patient may be trended within a night in order to make PAP titration decisions. After it is determined that the patient enters N2 sleep and CSR initiates, the PAP device may be inhibited from making pressure support interventions in order to control ventilation while a baseline sympathetic activation analysis is performed. In accordance with embodiments, this analysis period may last for 5-10 CSR cycles (e.g., 5-10 minutes) and/or until a baseline assessment of sympathetic activation via HRV is performed. After baseline assessment, the PAP device may optionally treat CSR with dynamic pressure support (DPS); and sympathetic activation via HRV assessment may continue.

In accordance with embodiments, when sympathetic activation is determined to increase into a clinically-significant degree (e.g., >+1 standard deviations (SD) over baseline measurement) while DPS is active, the system may detect this, and PSmax may be decreased until sympathetic activation is determined to be within the baseline range. In these embodiments, multiple baseline periods may be established in a given night. Comparison thresholds may be stored in the SSI. In accordance with some embodiments, the baseline measurement for sleep apnea (SA) may occur during non-N2 sleep (e.g., during quiet prone wakefulness prior to sleep onset and/or during slow wave sleep (SWS).

Embodiments of the present system may be integrated into various PAP solutions and may be operated in home settings as well as in institutional settings such as, hospitals, nursing homes, rehabilitation centers, etc. As operations may be automated, a clinician may not be necessary for operation of embodiments of the present system, rather a patient may employ one or more external sensors by attaching them to the body of the patient and operate the PAP device in accordance with embodiments of the present system and the PAP device may recognize this and communicate with the external sensors. Accordingly, the present system may greatly enhance user convenience and greatly reduce costs.

Further variations of the present system would readily occur to a person of ordinary skill in the art and are encompassed by the following claims.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming), and any combination thereof;
f) hardware portions may be comprised of one or both of analog and digital portions;
g) any of the disclosed devices, features and/or portions thereof may be combined together or separated into further portions unless specifically stated otherwise;
h) no specific sequence of acts or steps is intended to be required unless specifically indicated; and
i) the term "plurality of" an element includes two or more of the claimed element, and does not imply any particular range of number of elements; that is, a plurality of elements may be as few as two elements, and may include an immeasurable number of elements.

## Claims

1. A titration support system (100) for a positive air pressure, PAP, device (102), the system comprising:
at least one controller (120, 190, 194);
an external sensor (114) comprising at least an electrical impedance tomography, EIT, sensor, the external sensor (114) configured to:
determine whether the EIT sensor is coupled to skin of a user;
establish communication with the at least one controller (120, 190, 194) in response to determining that the external sensor (114) is coupled to the skin of the user;
sense a physiological parameter of the user and form corresponding sensor information;
determine whether cardiac output is diminished based upon the sensor information; and
transmit the sensor information including an indication of whether the cardiac output is diminished to the at least one controller (120, 190, 194);
the at least one controller (120, 190, 194) configured to:
control a flow generator (144) to provide therapeutic air assist to the user;
receive the sensor information from the external sensor;
determine whether the sensor information comprises the indication that the cardiac output is diminished;
begin auto titration to restore the cardiac output to homeopathic levels in response to determining that the cardiac output is diminished;
identify a pressure sensitivity of the user based on trending of the cardiac output mapped as a function on a control parameter of the PAP device (102); and
control the PAP device (102) to adapt PAP therapy based on the pressure sensitivity.

2. The titration support system (100) of claim 1, wherein the external sensor (114) is further configured to determine mean pulmonary arterial pressure, MPAP.

3. The titration support system (100) of claim 2, wherein the external sensor (114) determines that the cardiac output is diminished when the mean pulmonary arterial pressure, MPAP, is determined to be equal to or greater than 25 mm Hg.

4. The titration support system (100) of any of the preceding claims, wherein the cardiac output parameter is mean pulmonary arterial pressure, MPAP, and wherein the control parameter is expiratory positive airway pressure, EPAP.

5. The titration support system (100) of claim 1, wherein the external sensor (114) is further configured to sense sympathetic nervous system activity by measuring electrodermal activity, form corresponding sensor information, and transmit this sensor information to the at least one controller (120, 190, 194).

6. The titration support system (100) of claim 1, wherein the external sensor (114) determines that the cardiac output is diminished when increased sympathetic nervous system activity is detected.

7. The titration support system (100) of claim 1, wherein the external sensor (114) further comprises at least one of an electrocardiogram, ECG, a photoplethysmogram, PPG, a galvanic skin response, GSR, and a balistocardiogram, BCG, sensor.

8. The titration support system (100) of claim 1, wherein to restore the cardiac output to homeopathic levels, the at least one controller (120, 190, 194) is further configured control the flow generator (144) to decrease expiratory positive airway pressure, EPAP, by 1 cm H₂O every threshold time period until the cardiac output is restored to the homeopathic levels.

9. The titration support system (100) of claim 1, wherein the at least one controller (120, 190, 194) is further configured to determine the homeopathic levels during a titration period.

10. The titration support system (100) of claim 1, wherein the at least one controller (120, 190, 194) is further configured to determine a maximum pressure PMAX of therapeutic air to assist.

11. A computer program, which, when executed by the controller (120, 190, 194) of the titration support system (100) of any one of claims 1-10, causes the titration support system (100) to perform a method of providing titration support for a positive air pressure, PAP, device (102), the method comprising acts of:
determining whether an electrical impedance tomography, EIT, sensor is coupled to skin of a user;
establishing communication with at least one controller (120, 190, 194) in response to determining that the external sensor (114) is coupled to the skin of the user;
sensing a physiological parameter of the user and forming corresponding sensor information;
determining whether cardiac output is diminished based upon the sensor information; and
transmitting the sensor information including an indication of whether the cardiac output is diminished to the at least one controller (120, 190, 194);
controlling a flow generator (144) to provide therapeutic air assist to the user;
receiving the sensor information from the external sensor (114);
determining whether the sensor information comprises the indication that the cardiac output is diminished;
beginning auto titration to restore the cardiac output to homeopathic levels in response to determining that the cardiac output is diminished;
identifying a pressure sensitivity of the user based on trending of the cardiac output mapped as a function on a control parameter of the PAP device (102); and
controlling the PAP device (102) to adapt PAP therapy based on the pressure sensitivity.

12. The computer program of claim 11, the method comprising determining mean pulmonary arterial pressure, MPAP.

13. The computer program of claim 12, the method comprising determining that the cardiac output is diminished when the mean pulmonary arterial pressure, MPAP is determined to be equal to or greater than 25 mm Hg.

14. The computer program of any of the claims 10-13, wherein the cardiac output parameter is mean pulmonary arterial pressure, MPAP, and wherein the control parameter is expiratory positive airway pressure, EPAP.

15. A computer readable non-transitory medium having computer readable program code for operating on a computer for performing a method of providing titration support for a positive air pressure, PAP, device (102), the method comprising acts of:
determining whether an electrical impedance tomography, EIT, sensor is coupled to skin of a user;
establishing communication with at least one controller (120, 190, 194) in response to determining that the external sensor (114) is coupled to the skin of the user;
sensing a physiological parameter of the user and forming corresponding sensor information;
determining whether cardiac output is diminished based upon the sensor information; and
transmitting the sensor information including an indication of whether the cardiac output is diminished to the at least one controller (120, 190, 194);
controlling a flow generator (144) to provide therapeutic air assist to the user;
receiving the sensor information from the external sensor (114);
determining whether the sensor information comprises the indication that the cardiac output is diminished; and
beginning auto titration to restore the cardiac output to homeopathic levels in response to determining that the cardiac output is diminished;
identifying a pressure sensitivity of the user based on trending of the cardiac output mapped as a function on a control parameter of the PAP device (102); and
controlling the PAP device (102) to adapt PAP therapy based on the pressure sensitivity.

## Patentansprüche

1. Titrationsunterstützungssystem (100) für eine Vorrichtung mit positivem Luftdruck, PAP, (102), wobei das System umfasst:
mindestens eine Steuereinheit (120, 190, 194);
einen externen Sensor (114), der mindestens einen elektrischen Impedanztomographie-, EIT-Sensor, umfasst, wobei der externe Sensor (114) dazu konfiguriert ist:
zu bestimmen, ob der EIT-Sensor mit der Haut eines Benutzers gekoppelt ist;
eine Kommunikation mit der mindestens einen Steuereinheit (120, 190, 194) als Reaktion auf das Bestimmen aufzubauen, dass der externe Sensor (114) mit der Haut des Benutzers gekoppelt ist;
einen physiologischen Parameter des Benutzers abzutasten und entsprechende Sensorinformationen zu bilden;
basierend auf den Sensorinformationen zu bestimmen, ob die Herzleistung vermindert ist; und
die Sensorinformationen, die eine Angabe, ob die Herzleistung vermindert ist, beinhalten, an mindestens eine Steuereinheit (120, 190, 194) zu übertragen;
wobei die mindestens eine Steuereinheit (120, 190, 194) dazu konfiguriert ist:
einen Strömungsgenerator (144) zu steuern, um dem Benutzer therapeutische Luftunterstützung bereitzustellen;
die Sensorinformationen von dem externen Sensor zu empfangen;
zu bestimmen, ob die Sensorinformationen die Angabe umfassen, dass die Herzleistung vermindert ist;
Selbst-Titration zu beginnen, um die Herzleistung auf homöopathische Stufen als Reaktion auf das Bestimmen, dass die Herzleistung vermindert ist, wiederherzustellen;
eine Druckempfindlichkeit des Benutzers basierend auf einem Trend der Herzleistung zu identifizieren, die in Abhängigkeit von einem Steuerparameter der PAP-Vorrichtung (102) zugeordnet wird; und
die PAP-Vorrichtung (102) zu steuern, um die PAP-Therapie basierend auf der Druckempfindlichkeit anzupassen.

2. Titrationsunterstützungssystem (100) nach Anspruch 1, wobei der externe Sensor (114) weiter dazu konfiguriert ist, den mittleren Pulmonararteriendruck, MPAP, zu bestimmen.

3. Titrationsunterstützungssystem (100) nach Anspruch 2, wobei der externe Sensor (114) bestimmt, dass die Herzleistung vermindert ist, wenn bestimmt wird, dass der mittlere Pulmonararteriendruck, MPAP, gleich oder größer als 25 mm Hg ist.

4. Titrationsunterstützungssystem (100) nach einem der vorstehenden Ansprüche, wobei der Herzleistungsparameter der mittlere Pulmonararteriendruck, MPAP, ist, und wobei der Steuerparameter der exspiratorische Atemwegsüberdruck, EPAP, ist.

5. Titrationsunterstützungssystem (100) nach Anspruch 1, wobei der externe Sensor (114) weiter dazu konfiguriert ist, die Aktivität des sympathischen Nervensystems durch Messen elektrodermaler Aktivität abzutasten, entsprechende Sensorinformationen zu bilden und diese Sensorinformationen an die mindestens eine Steuereinheit (120, 190, 194) zu übertragen.

6. Titrationsunterstützungssystem (100) nach Anspruch 1, wobei der externe Sensor (114) bestimmt, dass die Herzleistung vermindert ist, wenn erhöhte Aktivität des sympathischen Nervensystems erfasst wird.

7. Titrationsunterstützungssystem (100) nach Anspruch 1, wobei der externe Sensor (114) weiter mindestens eines von einem Elektrokardiogramm-, EKG-, einem Photoplethysmogramm-, PPG-, einem galvanischen Hautreaktions-, GSR- und einem Balistokardiogramm-, BCG-Sensor umfasst.

8. Titrationsunterstützungssystem (100) nach Anspruch 1, wobei die mindestens eine Steuereinheit (120, 190, 194) zum Wiederherstellen der Herzleistung auf homöopathische Stufen weiter dazu konfiguriert ist, den Strömungsgenerator (144) zu steuern, um den exspiratorischen Atemwegsüberdruck, EPAP, in jedem Schwellenzeitraum um 1 cm H₂O zu senken, bis die Herzleistung auf die homöopathischen Stufen wiederhergestellt ist.

9. Titrationsunterstützungssystem (100) nach Anspruch 1, wobei die mindestens eine Steuereinheit (120, 190, 194) weiter dazu konfiguriert ist, die homöopathischen Stufen während eines Titrationszeitraums zu bestimmen.

10. Titrationsunterstützungssystem (100) nach Anspruch 1, wobei die mindestens eine Steuereinheit (120, 190, 194) weiter dazu konfiguriert ist, einen Maximaldruck PMAX der zu unterstützenden therapeutischen Luft zu bestimmen.

11. Computerprogramm, das, wenn es von der Steuereinheit (120, 190, 194) des Titrationsunterstützungssystems (100) nach einem der Ansprüche 1-10 ausgeführt wird, das Titrationsunterstützungssystem (100) veranlasst, ein Verfahren zum Bereitstellen von Titrationsunterstützung für eine Vorrichtung mit positivem Luftdruck (PAP) (102) durchzuführen, wobei das Verfahren Handlungen umfasst zum:
Bestimmen, ob ein elektrischer Impedanztomographie-, EIT-Sensor mit der Haut eines Benutzers gekoppelt ist;
Aufbauen einer Kommunikation mit der mindestens einen Steuereinheit (120, 190, 194) als Reaktion auf das Bestimmen, dass der externe Sensor (114) mit der Haut des Benutzers gekoppelt ist;
Abtasten eines physiologischen Parameters des Benutzers und Bilden entsprechender Sensorinformationen;
Bestimmen, ob die Herzleistung vermindert ist, basierend auf den Sensorinformationen; und
Übertragen der Sensorinformationen, die eine Angabe, ob die Herzleistung vermindert ist, beinhalten, an mindestens eine Steuereinheit (120, 190, 194);
Steuern eines Strömungsgenerators (144) zum Bereitstellen therapeutischer Luftunterstützung an den Benutzer;
Empfangen der Sensorinformationen von dem externen Sensor (114);
Bestimmen, ob die Sensorinformationen die Angabe umfassen, dass die Herzleistung vermindert ist;
Beginnen der Selbst-Titration, um die Herzleistung auf homöopathische Stufen als Reaktion auf das Bestimmen, dass die Herzleistung vermindert ist, wiederherzustellen;
Identifizieren einer Druckempfindlichkeit des Benutzers basierend auf einem Trend der Herzleistung, die in Abhängigkeit von einem Steuerparameter der PAP-Vorrichtung (102) zugeordnet wird; und
Steuern der PAP-Vorrichtung (102), um die PAP-Therapie basierend auf der Druckempfindlichkeit anzupassen.

12. Computerprogramm nach Anspruch 11, wobei das Verfahren das Bestimmen des mittleren Pulmonararteriendrucks (MPAP) umfasst.

13. Computerprogramm nach Anspruch 12, wobei das Verfahren das Bestimmen umfasst, dass die Herzleistung vermindert ist, wenn bestimmt wird, dass der mittlere Pulmonararteriendruck, MPAP, gleich oder größer als 25 mm Hg ist.

14. Computerprogramm nach einem der Ansprüche 10-13, wobei der Herzleistungsparameter der mittlere Pulmonararteriendruck, MPAP, ist, und wobei der Steuerparameter der exspiratorische Atemwegsüberdruck, EPAP, ist.

15. Computerlesbares, nichtflüchtiges Medium, das einen computerlesbaren Programmcode zum Betreiben auf einem Computer aufweist, zum Ausführen eines Verfahrens zum Bereitstellen einer Titrationsunterstützung für eine Vorrichtung mit positivem Luftdruck, PAP, (102), wobei das Verfahren Handlungen umfasst zum:
Bestimmen, ob ein elektrischer Impedanztomographie-, EIT-Sensor mit der Haut eines Benutzers gekoppelt ist;
Aufbauen einer Kommunikation mit der mindestens einen Steuereinheit (120, 190, 194) als Reaktion auf das Bestimmen, dass der externe Sensor (114) mit der Haut des Benutzers gekoppelt ist;
Abtasten eines physiologischen Parameters des Benutzers und Bilden entsprechender Sensorinformationen;
Bestimmen, ob die Herzleistung vermindert ist, basierend auf den Sensorinformationen; und
Übertragen der Sensorinformationen, die eine Angabe, ob die Herzleistung vermindert ist, beinhalten, an mindestens eine Steuereinheit (120, 190, 194);
Steuern eines Strömungsgenerators (144) zum Bereitstellen therapeutischer Luftunterstützung an den Benutzer;
Empfangen der Sensorinformationen von dem externen Sensor (114);
Bestimmen, ob die Sensorinformationen die Angabe umfassen, dass die Herzleistung vermindert ist; und
Beginnen der Selbst-Titration, um die Herzleistung auf homöopathische Stufen als Reaktion auf das Bestimmen, dass die Herzleistung vermindert ist, wiederherzustellen;
Identifizieren einer Druckempfindlichkeit des Benutzers basierend auf einem Trend der Herzleistung, die in Abhängigkeit von einem Steuerparameter der PAP-Vorrichtung (102) zugeordnet wird; und
Steuern der PAP-Vorrichtung (102), um die PAP-Therapie basierend auf der Druckempfindlichkeit anzupassen.

## Revendications

1. Système de support de titrage (100) pour un dispositif à pression d'air positive, PAP (102), le système comprenant :
au moins un dispositif de commande (120, 190, 194) ;
un capteur externe (114) comprenant au moins un capteur de tomographie par impédance électrique, EIT, le capteur externe (114) étant configuré pour :
déterminer si le capteur EIT est couplé à la peau d'un utilisateur ;
établir une communication avec le au moins un dispositif de commande (120, 190, 194) en réponse à la détermination du fait que le capteur externe (114) est couplé à la peau de l'utilisateur ;
détecter un paramètre physiologique de l'utilisateur et former des informations de capteur correspondantes ;
déterminer si le débit cardiaque est diminué sur la base des informations de capteur ; et
transmettre les informations de capteur incluant une indication du fait que le débit cardiaque est ou non diminué au au moins un dispositif de commande (120, 190, 194) ;
le au moins un dispositif de commande (120, 190, 194) étant configuré pour :
commander un générateur de flux (144) pour fournir une assistance d'air thérapeutique à l'utilisateur ;
recevoir les informations de capteur du capteur externe ;
déterminer si les informations de capteur comprennent l'indication du fait que le débit cardiaque est diminué ;
commencer l'autotitrage pour rétablir le débit cardiaque aux niveaux homéopathiques en réponse à la détermination du fait que le débit cardiaque est diminué ;
identifier une sensibilité à la pression de l'utilisateur sur la base de la tendance du débit cardiaque cartographié en fonction d'un paramètre de commande du dispositif PAP (102) ; et
commander le dispositif PAP (102) pour adapter la thérapie PAP sur la base de la sensibilité à la pression.

2. Système de support de titrage (100) selon la revendication 1, dans lequel le capteur externe (114) est en outre configuré pour déterminer la pression artérielle pulmonaire moyenne, MPAP.

3. Système de support de titrage (100) selon la revendication 2, dans lequel le capteur externe (114) détermine que le débit cardiaque est diminué lorsque la pression artérielle pulmonaire moyenne, MPAP, est déterminée comme étant égale ou supérieure à 25 mmHg.

4. Système de support de titrage (100) selon l'une quelconque des revendications précédentes, dans lequel le paramètre de débit cardiaque est la pression artérielle pulmonaire moyenne, MPAP, et dans lequel le paramètre de commande est la pression positive expiratoire des voies aériennes, EPAP.

5. Système de support de titrage (100) selon la revendication 1, dans lequel le capteur externe (114) est en outre configuré pour détecter l'activité du système nerveux sympathique en mesurant l'activité électrodermique, former des informations de capteur correspondantes et transmettre ces informations de capteur au au moins un dispositif de commande (120, 190, 194).

6. Système de support de titrage (100) selon la revendication 1, dans lequel le capteur externe (114) détermine que le débit cardiaque est diminué lorsqu'une activité accrue du système nerveux sympathique est détectée.

7. Système de support de titrage (100) selon la revendication 1, dans lequel le capteur externe (114) comprend en outre au moins l'un parmi un électrocardiogramme, ECG, un photopléthysmogramme, PPG, une réponse galvanique de la peau, GSR, et un balistocardiogramme, BCG.

8. Système de support de titrage (100) selon la revendication 1, dans lequel, pour restaurer le débit cardiaque à des niveaux homéopathiques, le au moins un dispositif de commande (120, 190, 194) est en outre configuré pour commander le générateur de débit (144) pour diminuer la pression positive expiratoire des voies aériennes, EPAP, de 1 cm H₂O à chaque période de temps seuil jusqu'à ce que le débit cardiaque soit restauré aux niveaux homéopathiques.

9. Système de support de titrage (100) selon la revendication 1, dans lequel le au moins un dispositif de commande (120, 190, 194) est en outre configuré pour déterminer les niveaux homéopathiques pendant une période de titrage.

10. Système de support de titrage (100) selon la revendication 1, dans lequel le au moins un dispositif de commande (120, 190, 194) est en outre configuré pour déterminer une pression maximale PMAX d'air thérapeutique à assister.

11. Programme informatique qui, lorsqu'il est exécuté par le dispositif de commande (120, 190, 194) du système de support de titrage (100) selon l'une quelconque des revendications 1-10, amène le système de support de titrage (100) à effectuer un procédé de fourniture de support de titrage pour un dispositif à pression d'air positive, PAP (102), le procédé comprenant les actes consistant à :
déterminer si un capteur de tomographie par impédance électrique, EIT, est couplé à la peau d'un utilisateur ;
établir une communication avec au moins un dispositif de commande (120, 190, 194) en réponse à la détermination du fait que le capteur externe (114) est couplé à la peau de l'utilisateur ;
détecter un paramètre physiologique de l'utilisateur et former des informations de capteur correspondantes ;
déterminer si le débit cardiaque est diminué sur la base des informations de capteur ; et
transmettre les informations de capteur incluant une indication du fait que le débit cardiaque est ou non diminué au au moins un dispositif de commande (120, 190, 194) ;
commander un générateur de flux (144) pour fournir une assistance d'air thérapeutique à l'utilisateur ;
recevoir les informations de capteur du capteur externe (114) ;
déterminer si les informations de capteur comprennent l'indication du fait que le débit cardiaque est diminué ;
commencer l'autotitrage pour rétablir le débit cardiaque aux niveaux homéopathiques en réponse à la détermination du fait que le débit cardiaque est diminué ;
identifier une sensibilité à la pression de l'utilisateur sur la base de la tendance du débit cardiaque cartographié en fonction d'un paramètre de commande du dispositif PAP (102) ; et
commander le dispositif PAP (102) pour adapter la thérapie PAP sur la base de la sensibilité à la pression.

12. Programme informatique selon la revendication 11, le procédé comprenant la détermination de la pression artérielle pulmonaire moyenne, MPAP.

13. Programme informatique selon la revendication 12, le procédé comprenant la détermination du fait que le débit cardiaque est diminué lorsque la pression artérielle pulmonaire moyenne, MPAP, est déterminée comme étant égale ou supérieure à 25 mmHg.

14. Programme informatique selon l'une quelconque des revendications 10-13, dans lequel le paramètre de débit cardiaque est la pression artérielle pulmonaire moyenne, MPAP, et dans lequel le paramètre de commande est la pression positive expiratoire des voies aériennes, EPAP.

15. Support non transitoire lisible par ordinateur présentant un code de programme lisible par ordinateur destiné à fonctionner sur un ordinateur pour effectuer un procédé de fourniture de support de titrage pour un dispositif à pression d'air positive, PAP (102), le procédé comprenant les actes consistant à :
déterminer si un capteur de tomographie par impédance électrique, EIT, est couplé à la peau d'un utilisateur ;
établir une communication avec au moins un dispositif de commande (120, 190, 194) en réponse à la détermination du fait que le capteur externe (114) est couplé à la peau de l'utilisateur ;
détecter un paramètre physiologique de l'utilisateur et former des informations de capteur correspondantes ;
déterminer si le débit cardiaque est diminué sur la base des informations de capteur ; et
transmettre les informations de capteur incluant une indication du fait que le débit cardiaque est ou non diminué au au moins un dispositif de commande (120, 190, 194) ;
commander un générateur de flux (144) pour fournir une assistance d'air thérapeutique à l'utilisateur ;
recevoir les informations de capteur du capteur externe (114) ;
déterminer si les informations de capteur comprennent l'indication du fait que le débit cardiaque est diminué ; et
commencer l'autotitrage pour rétablir le débit cardiaque aux niveaux homéopathiques en réponse à la détermination du fait que le débit cardiaque est diminué ;
identifier une sensibilité à la pression de l'utilisateur sur la base de la tendance du débit cardiaque cartographié en fonction d'un paramètre de commande du dispositif PAP (102) ; et
commander le dispositif PAP (102) pour adapter la thérapie PAP sur la base de la sensibilité à la pression.
